(19) Europäisches Patentamt
European Patent Office
Office européen des brevets

(11) EP 3 020 726 A1

(12) # EUROPÄISCHE PATENTANMELDUNG

(43) Veröffentlichungstag:
**18.05.2016 Patentblatt 2016/20**

(51) Int Cl.:
***C07K 14/47*** *(2006.01)* ***B01D 15/12*** *(2006.01)*
***B01D 15/38*** *(2006.01)*

(21) Anmeldenummer: **14192831.7**

(22) Anmeldetag: **12.11.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Pentracor GmbH**
**16761 Hennigsdorf (DE)**

(72) Erfinder:
- **Sheriff, Ahmed**
  **10713 Berlin (DE)**
- **Vogt, Birgit**
  **13353 Berlin (DE)**
- **Mattecka, Stephan**
  **13053 Berlin (DE)**

(74) Vertreter: **Arth, Hans-Lothar**
**ABK Patent Attorneys**
**Jasminweg 9**
**14052 Berlin (DE)**

(54) **Verwendung einer Citrat-Lösung zur affinitätschromatographischen Aufreinigung von CRP mittels Phosphocholin und dessen Derivaten**

(57) Die vorliegende Erfindung betrifft die Verwendung einer Citrat-Lösung zur affinitätschromatographischen Entfernung von C-reaktivem Protein (CRP) aus biologischen Flüssigkeiten, wobei die affinitätschromatographische Entfernung von CRP durch (eine $Ca^{2+}$-abhängige) Bindung von CRP an ein mit ω-Phosphonooxyalkylammoniumgruppen und/oder mit ω-Ammoniumalkoxy-hydroxyphosphoryloxygruppen funktionalisiertes Säulenmaterial erfolgt.

**Fig. 1**

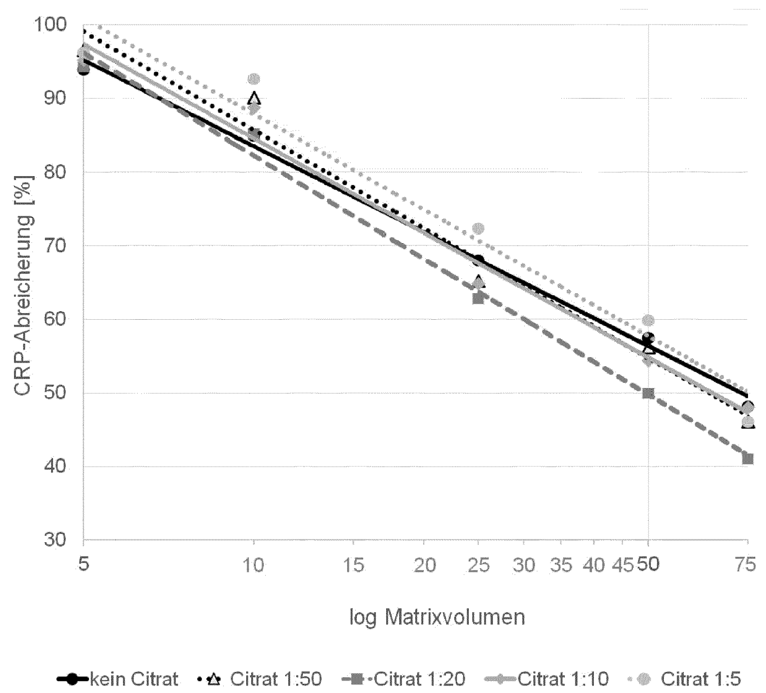


EP 3 020 726 A1

## Beschreibung

Die vorliegende Erfindung betrifft die Verwendung einer Citrat-Lösung zur affinitätschromatographischen Entfernung von C-reaktivem Protein (CRP) aus biologischen Flüssigkeiten, wobei die affinitätschromatographische Entfernung von CRP durch (eine $Ca^{2+}$-abhängige) Bindung von CRP an ein mit ω-Phosphonooxyalkylammoniumgruppen und/oder mit ω-Ammoniumalkoxy-hydroxy-phosphoryloxygruppen funktionalisiertes Säulenmaterial erfolgt.

## Hintergrund der Erfindung

Nach Angaben der Weltgesundheitsorganisation (*World Health Organization,* WHO) starben im Jahr 2008 circa 17.000.000 Menschen an kardiovaskulären Erkrankungen. Damit sind kardiovaskuläre Erkrankungen die häufigste Todesursache unter den nichtübertragbaren Krankheiten und verantwortlich für ungefähr ein Drittel der jährlich weltweit auftretenden Todesfälle. Nach Schätzungen wird diese Zahl bis zum Jahr 2030 auf ungefähr 23.000.000 Todesfälle pro Jahr ansteigen.

Somit sind und bleiben kardiovaskuläre Erkrankungen nicht nur die Haupttodesursache weltweit, sondern verursachen auch enorme medizinische Kosten für die nationalen Gesundheitssysteme und Krankenkassen. Zwei der häufigsten und schädlichsten Erscheinungen kardiovaskulärer Erkrankungen sind das Auftreten von Arteriosklerose und Thrombose, welche wiederum unter anderem ursächlich für Herzinfarkte und Schlaganfälle sind.

In den letzten Jahren wurden große Fortschritte in der Behandlung von kardiovaskulären Erkrankungen erzielt. Dieser Fortschritt wurde nicht nur durch wachsende Erkenntnisse hinsichtlich der Krankheits-auslösenden Mechanismen ermöglicht, sondern auch durch die frühzeitige Identifizierung von Risiko-Patienten. Tatsächlich sind die Identifizierung von Erkrankungsrisiken und deren frühzeitige Behandlung wichtige Merkmale der modernen medizinischen Praxis. In den letzten 25 Jahren wurde eine Vielzahl an Faktoren und klinischen Parametern identifiziert, die entweder mit dem gegenwärtigen Zustand der Erkrankung oder mit der zukünftigen Wahrscheinlichkeit für eine kardiovaskuläre Erkrankung korrelieren. Solche Risikofaktoren können messbare biochemische oder physiologische Parameter wie z.B. Serum-Cholesterin-, HDL-, LDL-, und Fibrinogen-Spiegel sein aber auch Verhaltensmuster wie z.B. Übergewicht und Rauchen beinhalten. In Fällen, in denen ein Risikofaktor nicht lediglich auf eine Erkrankung oder deren Entstehung hinweist, sondern tatsächlich ursächlich an deren Entstehung beteiligt ist, kann eine therapeutische Beeinflussung dieses Risikofaktors den Verlauf der Erkrankung beeinflussen bzw. das Risiko ihrer Entstehung verringern.

C-reaktives Protein (CRP) ist als Akut-Phase-Protein Teil des angeborenen Immunsystems und wird in der Leber im Zuge von Entzündungsreaktionen gebildet und ins Blut abgegeben. Die Bildung von CRP wird hierbei in erster Linie durch Cytokine induziert, die im Rahmen einer akuten oder chronischen Entzündungsreaktion exprimiert werden. Der stärkste Stimulus zur Bildung von CRP ist hierbei das Interleukin-6 (IL-6). Deshalb sind die Spiegel von CRP ebenso wie von IL-6 im Blut Indikatoren für eine lokale oder systemische Entzündungsreaktion. Chronische Entzündungen werden als eine der zugrundeliegenden und unterstützenden pathologischen Erscheinungen bei kardiovaskulären Erkrankungen vermutet. Hierbei wird zunehmend davon ausgegangen, dass CRP nicht nur prädikativ für kardiovaskuläre Erkrankungen ist, sondern auch kausal an deren Entstehung beteiligt ist bzw. deren Verlauf beeinflussen kann.

Yeh (Clin Cardiol., 2005, 28: 408-412) zeigt, dass der CRP-Spiegel verwendet werden kann, um das kardiovaskuläre Erkrankungsrisiko vorauszusagen, CRP außerdem ein Indikator für Entzündungsreaktionen ist, und dass Entzündungen alle Stadien der Atherosklerose befördern. Zoccali et al., (Semin. Nephrol., 2005, 25: 358-362) zeigen, dass der CRP-Spiegel prädiktiv für das kardiovaskuläre Mortalitätsrisiko bei Patienten mit Nierenerkrankung im Endstadium ist. Nach Nurmohamed et al., (Neth. J. Med., 2005, 63: 376-381) ist der CRP-Spiegel prädiktiv für das kardiovaskuläre Mortalitätsrisiko bei Hämodialysepatienten.

Sola et al., (J. Card. Fail., 2005, 11: 607-612) konnten zeigen, dass Statintherapien dazu genutzt werden können, die Menge an CRP zu senken und so die durch kardiovaskuläre Erkrankung hervorgerufene Mortalität und Morbidität reduziert wird. Diese Therapieform genügt aber nicht, um die hohen CRP-Mengen (bis zu 1000fach über den Normalwerten), welche nach einem Herzinfarkt entstehen, oder die hohen CRP-Mengen im Blut von Dialysepatienten signifikant zu reduzieren.

Der Normalwert für CRP im Blut von Menschen variiert von Person zu Person, liegt aber im Median bei ungefähr 0,8 mg CRP pro Liter Blut, kann aber im Fall von akuten oder chronischen Entzündungsreaktionen (z.B. bakterielle Infektionen, Atherosklerose, nach einem Herzinfarkt) auf deutlich über 100 mg CRP pro Liter Blut steigen. Da die Halbwertszeit von CRP im Blut (ca. 19 Stunden) konstant und damit unabhängig vom gesundheitlichen Zustand des Patienten ist, ist allein die Syntheserate von CRP für die Regulation des CRP-Spiegels im Blut verantwortlich (Pepys & Hirschfield, J. Clin. Invest., 2003, 111: 1805-1812). Die stark erhöhte Synthese an CRP bei akuten pathologischen Konditionen stellt folglich besondere Anforderungen an therapeutische Ansätze zur CRP-Entfernung von Patienten (Risikopatienten oder Akut-Patienten), da eine erhebliche Menge an CRP entfernt werden muss, um den CRP-Spiegel im Blut auf Normalwerte zu senken.

Folglich besteht ein zunehmendes Interesse an therapeutischen Verfahren zur Reduzierung des CRP-Spiegels

im Blut von Patienten. Aufgrund der klinischen Signifikanz von CRP besteht zudem ein Interesse an effektiven Methoden zur Aufreinigung von CRP, um anschließend das aufgereinigte CRP in weiteren Experimenten, z.B. zur Untersuchung seiner molekularen Funktion, zu verwenden.

WO 2004/076486 offenbart eine Methode zur Inhibition immunologischer, inflammatorischer und/oder pathophysiologischer Antworten durch Verabreichung von CRP-bindenden Molekülen an Patienten mit einem erhöhten CRP-Spiegel. Nicht offenbart wird hingegen die extrakorporale Behandlung von biologischen Flüssigkeiten zur Entfernung von CRP aus den genannten biologischen Flüssigkeiten.

WO 90/12632 offenbart eine Methode und Vorrichtung zur extrakorporalen Behandlung von biologischen Flüssigkeiten mit dem Ziel der Entfernung von CRP sowie von anti-Phosphocholin-Antikörpern aus diesen biologischen Flüssigkeiten zur Behandlung von Krebs. Die hierfür verwendete Phosphocholin-haltige Matrix kann z.B. aus Kieselerde, Sepharose, Acrylbeads oder Agarose bestehen, wobei durch das enthaltene Phosphocholin sowohl CRP als auch anti-Phosphocholin-Antikörper gebunden werden.

WO 2007/076844 offenbart eine Methode durch eine extrakorporale CRP-Entfernung aus dem Blutplasma mittels Apherese das Risiko für einen Patienten (verursacht durch einen erhöhten CRP-Spiegel im Blut) zu verringern. Gemäß der Erfindung wird hierzu eine Säule mit enthaltener Matrix verwendet, an die Phosphocholin-Derivate gebunden sind um CRP zu binden und aus dem Plasma zu entfernen und so Autoimmunkrankheiten, kardiovaskuläre Erkrankungen, Diabetes sowie Niereninsuffizienz zu behandeln und/oder ihnen vorzubeugen.

Slagman et al. (Blood Purif., 2011, 31: 9) demonstrieren am Schweine-Modell die erfolgreiche Reduzierung des CRP-Spiegels im Blut mittels extrakorporaler Apherese nach einem Herzinfarkt.

Ein Problem, dass bei der extrakorporalen Verwendung von Blut und Blutplasma auftritt, sei es bei analytischen oder präparativen Anwendungen aber auch bei Dialyse oder Apherese, ist die einsetzende Blutgerinnung, die sowohl die Verwendung selbst behindert oder unmöglich macht, aber ebenso verwendete Geräte verstopfen bzw. verunreinigen kann. Aus diesem Grund werden dem Blut oder Blutplasma nach der Entfernung aus dem menschlichen oder tierischen Körper unmittelbar Gerinnungshemmstoffe (so genannte Antikoagulationsmittel) zugesetzt.

Eine Möglichkeit zur Hemmung der Blutgerinnung ist die Verabreichung von $Ca^{2+}$-Chelatoren wie z.B. EDTA (Ethylendiamintetraessigsäure), Oxalat und Citrate. Calcium wird während der Blutgerinnung als Cofaktor einiger Gerinnungsfaktoren benötigt. Mithilfe von $Ca^{2+}$-Chelatoren wird dem Blut oder Blutplasma Calcium entzogen und die Gerinnung somit gehemmt. Hier liegt jedoch ein wesentliches Problem für affinitätschromatographische Verfahren zur Entfernung von CRP mittels Phosphocholin oder Phospoethanolamin bzw. Phosphocholin-Derivaten oder Phospoethanolamin-Derivaten. Da die Bindung von CRP an Phosphocholin bzw. an dessen Derivate $Ca^{2+}$ benötigt (Black et al., Journal of Biological Chemistry, 2004, 279: 48487; Thompson et al., Structure, 1999, 7 (2): 169-177), war die einhellige Meinung im Stand der Technik, dass eine Gabe von $Ca^{2+}$-Chelatoren während der Bindung von CRP an Phosphocholin unbedingt zu vermeiden sei (siehe z.B. WO 90/12632). Somit ist die Verwendung von citrathaltigen Bindungspuffern während der affinitätschromatographischen Bindung von CRP an Phosphocholin laut dem Stand der Technik völlig ungeeignet. Citrathaltige Lösungen eigneten sich jedoch (ähnlich wie die Gabe von EDTA) als Elutionspuffer, also um das gebundene CRP wieder vom Phosphocholin zu lösen.

Eine Möglichkeit zur $Ca^{2+}$-unabhängigen Antikoagulation besteht in der Verabreichung von Heparin. Heparin ist eine körpereigene Substanz, welche durch Mastzellen produziert wird und hemmend auf die Gerinnungskaskade wirkt. Durch die Bindung von Heparin an Antithrombin II, ein im Blut zirkulierender Proteaseinhibitor, der aktivierte Gerinnungsfaktoren wie Thrombin und Faktor Xa inaktivieren kann, wird eine Konformationsänderung des Antithrombins II ausgelöst. Dadurch wird die Antithrombin II-vermittelte Inaktivierung von Gerinnungsfaktoren um das 2000-fache beschleunigt und. die Blutgerinnung kommt zum Erliegen. Folglich wird in Verfahren zur affinitätschromatographischen Entfernung von CRP aus Blut oder Blutplasma mittels Phosphocholin und Phosphocholin-Derivaten bzw. Phospoethanolamin und Phospoethanolamin-Derivaten bisher ausschließlich Heparin als Antikoagulationsmittel verwendet.

Dies birgt jedoch einige Nachteile, da es bei der Verwendung von Heparin, gerade bei unfraktioniertem Heparin, neben Blutungen auch zu einer Immunreaktion, der heparininduzierten Thrombozytopenie (HIT), kommen kann. Dabei induziert das Heparin venöse und arterielle Thrombosen. Bei Schlaganfallpatienten, bei denen die Anwendung einer CRP-Apherese ebenfalls möglich ist, liegt das Risiko einer HIT bei dem Erhalt von unfraktioniertem Heparin bei ca. 3 %. Zudem ist Heparin nicht zur Verwendung mit zentrifugalen Zellseparatoren zugelassen, welche oft zur Trennung von Blut in die zellulären Bestandteile und das Blutplasma verwendet werden.

Aufgabe der vorliegenden Erfindung ist es, ein Antikoagulanz für eine affinitätschromatographische Entfernung von CRP bereitzustellen, welches die Heparinbedingten Probleme aus dem Stand der Technik vermeidet und zudem die $Ca^{2+}$-abhängige Bindung von CRP an Phosphocholin bzw. Phosphoethanolamin und deren Derivate nicht beeinträchtigt.

Diese Aufgabe wird durch die Lehre der unabhängigen Ansprüche gelöst. Weitere vorteilhafte Ausgestaltungen ergeben sich aus der Beschreibung, den Beispielen und den anhängigen Patentansprüchen.

Überraschend wurde gefunden, dass durch die Verwendung einer Citrat-Lösung zur affinitätschromatographischen Entfernung von CRP aus biologischen Flüssigkeiten mittels eines mit ω-Phosphonooxyalkylammoniumgruppen

und/oder mit ω-Ammoniumalkoxy-hydroxy-phosphoryloxygruppen funktionalisierten Säulenmaterials nicht nur überraschend eine effiziente Entfernung von CRP aus biologischen Flüssigkeiten möglich ist, sondern hierdurch auch die Probleme aus dem Stand der Technik beseitigt werden.

**Beschreibung der Erfindung**

Die vorliegende Erfindung betrifft die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials.

Der Begriff **"CRP",** wie hierin verwendet, ist gleichbedeutend mit "C-reaktives Protein". Er bezieht sich hierin bevorzugt auf das humane C-reaktive Protein.

Der Begriff **"affinitätschromatographische Entfernung von CRP",** wie hierin verwendet, bedeutet, dass die Entfernung von CRP durch eine spezifische Bindung zwischen CRP und Bestandteilen des Mittels zur Entfernung von CRP geschieht. Man kann in diesem Zusammenhang auch von einer "affinitätschromatographischen Entfernung von CRP" oder von einer "selektiven Entfernung von CRP" sprechen. Eine derartige spezifische Bindung zwischen CRP und einem entsprechend funktionalisierten Säulenmaterial beruht auf den strukturellen Eigenschaften des CRP-Proteins und können beispielsweise die charakteristische Bindung von CRP an Phosphocholin oder die Bindung von CRP an Antikörper, die gegen ein Epitop des CRP gerichtet sind, umfassen. Die selektive oder molekülspezifische Entfernung von CRP beinhaltet, dass CRP mit höherer Affinität an das funktionalisierte Säulenmaterial bindet als an andere Bestandteile der biologischen Flüssigkeiten. Der Begriff "selektiv" in Bezug zur Entfernung von CRP, wie in der vorliegenden Anmeldung verwendet, bedeutet daher, dass das Verhältnis von entferntem CRP zur Menge des in der biologischen Flüssigkeit enthaltenen CRP mindestens dreimal so hoch ist wie das jeweilige Verhältnis von anderen entfernten Substanzen zur Menge der in der biologischen Flüssigkeit enthaltenen Substanz. Der Begriff "selektiv" in Bezug zur Entfernung von CRP, wie in der vorliegenden Anmeldung verwendet, bedeutet jedoch nicht, dass ausschließlich CRP entfernt wird. Hier ist es für den Fachmann offensichtlich, dass bei einer derartigen affinitätschromatographischen Entfernung von CRP zu einem gewissen Grad zwangsläufig auch andere Substanzen (ungewollt) an das Säulenmaterial binden können und somit auch zu einem gewissen Grad mit entfernt werden. Gibt es z.B. bei einem Protein zu einem gewissen Grad eine strukturelle Übereinstimmung mit der Struktur des CRPs, die für die spezifische Bindung an die ω-Phosphonooxyalkylammoniumgruppen und/oder mit ω-Ammoniumalkoxy-hydroxy-phosphoryloxygruppen verantwortlich ist, so wird auch dieses strukturähnliche Protein zu einem bestimmten Grad gebunden. Ein Beispiel wären z.B. Antikörper, die gegen Phosphocholin gerichtet sind und daher bei einem Säulenmaterial, das mit Phosphocholin funktionalisiert wurde, ebenfalls gebunden werden können. Eine andere Möglichkeit besteht in der nie vollständig vermeidbaren unspezifischen Bindung von Bestandteilen der biologischen Flüssigkeit an z.B. Matrixsubstratmaterialien.

Wie bereits erwähnt, ist die Bindung von CRP an Phosphocholin oder an dessen Derivate bzw. an Phospoethanolamin oder an dessen Derivate abhängig von der Gegenwart von $Ca^{2+}$ und somit ist auch die Bindung von CRP an ω-Phosphonooxyalkylammoniumgruppen und/oder mit ω-Ammoniumalkoxy-hydroxy-phosphoryloxygruppen, die in bzw. an einem Säulenmaterial immobilisiert wurden, ebenfalls abhängig von der Gegenwart von $Ca^{2+}$.

Daher betrifft die vorliegende Erfindung ebenso die Verwendung einer Citrat-Lösung zur affinitätschromatographischen Entfernung von CRP aus biologischen Flüssigkeiten, wobei die affinitätschromatographische Entfernung von CRP durch (eine $Ca^{2+}$-abhängige) Bindung von CRP an ein mit ω-Phosphonooxyalkylammoniumgruppen und/oder mit ω-Ammoniumalkoxy-hydroxy-phosphoryloxygruppen funktionalisiertes Säulenmaterial erfolgt.

Natürlich bedeutet die erfindungsgemäße Verwendung einer Citrat-Lösung zur affinitätschromatographischen Entfernung von CRP aus biologischen Flüssigkeiten, wobei die affinitätschromatographische Entfernung von CRP durch (eine $Ca^{2+}$-abhängige) Bindung von CRP an ein mit ω-Phosphonooxyalkylammoniumgruppen und/oder mit ω-Ammoniumalkoxy-hydroxy-phosphoryloxygruppen funktionalisiertes Säulenmaterial erfolgt, dass die Citrat-Lösung vor der CRP-Entfernung mit der biologischen Flüssigkeit, aus der CRP entfernt werden soll, vermischt wird. Die Citrat-Lösung ist also während der gewünschten Bindung von CRP an die ω-Phosphonooxyalkylammoniumgruppen und/oder ω-Ammoniumalkoxy-hydroxy-phosphoryloxygruppen des Säulenmaterials anwesend. Man könnte also in Anlehnung an Fachbegriffe aus dem Bereich der Affinitätschromatographie auch davon sprechen, dass die Citrat-Lösung als "Bindungspuffer" verwendet wird.

Es bedeutet hingegen **nicht,** dass die Citrat-Lösung als "Elutionslösung" (oder als "Elutionspuffer") und somit zur Entfernung des CRPs von dem mit ω-Phosphonooxyalkylammoniumgruppen und/oder mit ω-Ammoniumalkoxy-hydroxy-phosphoryloxygruppen funktionalisiertes Säulenmaterial verwendet wird, also um das Säulenmaterial zu regenerieren.

Der Begriff **"Bindungspuffer"** (auch als "Bindungslösung" bezeichnet), wie hierin verwendet, bezeichnet bei einem affinitätschromatographischen Verfahren zur Entfernung einer Substanz (hier die selektive und $Ca^{2+}$-abhängige Entfernung von CRP) aus einer Probe (hier biologische Flüssigkeiten wie z.B. Blut oder Blutplasma) eine Lösung, die der Probe zugesetzt wird und dann zusammen mit der Probe auf das Säulenmaterial zur Entfernung der Substanz (hier

ein mit ω-Phosphonooxyalkylammoniumgruppen und/oder mit ω-Ammoniumalkoxy-hydroxy-phosphoryloxygruppen funktionalisiertes Säulenmaterial) aufgetragen wird. Durch den Bindungspuffer sollen adäquate Bedingungen für die spezifische Bindung der zu entfernenden Substanz an das Säulenmaterial gewährleistet werden.

Der Begriff **"Elutionspuffer"** (auch als "Elutionslösung" bezeichnet), wie hierin verwendet, bezeichnet bei einem affinitätschromatographischen Verfahren zur Entfernung einer Substanz (hier die selektive und $Ca^{2+}$-abhängige Entfernung von CRP) aus einer Probe (hier biologische Flüssigkeiten wie z.B. Blut oder Blutplasma) eine Lösung, die nach der Auftragung der Probe auf das Säulenmaterial und der erfolgten spezifischen Bindung der zu entfernenden Substanz an das Säulenmaterial, aufgetragen wird, um diese spezifische Bindung wieder zu lösen und somit die zu entfernende Substanz wieder vom dem Säulenmaterial zu lösen (bzw. zu eluieren). Im Gegensatz zum Bindungspuffer sollen also durch den Elutionspuffer Bedingungen im Säulenmaterial geschaffen werden, die eben keine Bindung der zu entfernenden Substanz ermöglichen, sondern diese im Gegenteil sogar verhindern.

Die vorliegende Erfindung betrifft daher auch die Verwendung einer Citrat-Lösung zur affinitätschromatographischen Entfernung von CRP aus biologischen Flüssigkeiten, wobei die affinitätschromatographische Entfernung von CRP durch (eine $Ca^{2+}$-abhängige) Bindung von CRP an ein mit ω-Phosphonooxyalkylammoniumgruppen und/oder mit ω-Ammoniumalkoxy-hydroxy-phosphoryloxygruppen funktionalisiertes Säulenmaterial erfolgt,
wobei die Citrat-Lösung als Bindungspuffer (oder Bindungslösung) bei der affinitätschromatographischen Entfernung von CRP aus biologischen Flüssigkeiten dient.

Der Betriff **"biologische Flüssigkeit",** wie hierin verwendet, bezieht sich auf wässrige Lösungen, die in Säugetieren und vorzugsweise Menschen auftreten, wie z.B. Zerebrospinalflüssigkeit, Peritonealflüssigkeit, Pleura-Flüssigkeit, Aszites-Flüssigkeit, Blut, Blutplasma, Leber-Extrakte und Interstitialflüssigkeit. Die vorliegende Erfindung bezieht sich natürlich auf biologische Flüssigkeiten, die CRP enthalten.

Die vorliegende Erfindung ist somit ebenfalls gerichtet auf die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, wobei es sich bei den biologischen Flüssigkeiten um Blut oder Blutplasma handelt.

Anders ausgedrückt, ist die vorliegende Erfindung ebenfalls gerichtet auf die Verwendung einer Citrat-Lösung zur affinitätschromatographischen Entfernung von CRP aus Blut und/oder Blutplasma mittels eines mit ω-Phosphonooxyalkylammoniumgruppen und/oder mit ω-Ammoniumalkoxy-hydroxy-phosphoryloxygruppen funktionalisierten Säulenmaterials.

## Citrat-Lösungen

Der Begriff **"Citrat",** wie hierin verwendet, bezieht sich auf das Citrat-Anion, also das Salz der Zitronensäure, oder anders ausgedrückt ein organisches Tricarboxylat der folgenden chemischen Formel:

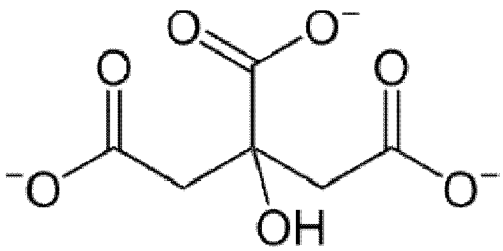

Das Citrat kann in verschiedenen Formen (bzw. Verbindungen) auftreten, also z.B. als Zitronensäure (in ein bis dreifach protonierter Form), als Salz der Zitronensäure in Kombination mit anderen (anders als $H^+$) anorganischen Kationen (z.B. als Metallsalz zusammen mit Metallkationen oder als Ammoniumsalz zusammen mit Ammoniumionen), aber auch als partieller Citratester. In diesem Zusammenhang wird hierin auch der Begriff **"Citrat-Verbindung"** verwendet.

Wird das Salz der Zitronensäure, also das Citrat-Anion, mit einem anorganischen Kation komplexiert, spricht man hierin auch von **"Citrat-Salz"** als spezielle Form der Citrat-Verbindung.

Der Begriff **"Citrat-Lösung",** wie hierin verwendet, umfasst folglich wässrige Lösungen die zumindest eine Citrat-Verbindung enthalten. Detailliertere Angaben zur Zusammensetzung der Citrat-Lösung werden weiter unten gemacht.

Erfindungsgemäß wird jedoch bevorzugt, wenn die Citrat-Konzentration in der Citrat-Lösung nicht unter 1 mM, bevorzugt nicht unter 1,1 mM, bevorzugt nicht unter 1,2 mM, weiter bevorzugt nicht unter 1,3 mM, noch weiter bevorzugt nicht unter 1,4 mM und am meisten bevorzugt nicht unter 1,5 mM liegt.

Zudem wird erfindungsgemäß bevorzugt, wenn die Citrat-Verbindung(en) ein wesentlicher Bestandteil der

erfindungsgemäßen Citrat-Lösung ist. Hierbei bedeutet "wesentlicher Bestandteil", dass die Summe der molaren Konzentrationen aller Citrat-Verbindungen in der Citrat-Lösung verglichen mit den sonstigen Verbindungen innerhalb der Citrat-Lösung, ausgenommen jedoch Wasser, unter den drei höchsten Konzentrationen liegt, die in der Citrat-Lösung auftreten.

Die vorliegende Erfindung bezieht sich daher auch auf die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, wobei die Citrat-Lösung mindestens eine der Citrat-Verbindungen aus der Gruppe umfassend oder bestehend aus Zitronensäure, Natriumdihydrogencitrat, Dinatriumhydrogencitrat, Trinatriumcitrat, Trinatriumcitrat Dihydrat, Kaliumdihydrogencitrat, Dikaliumhydrogencitrat, Trikaliumcitrat, Lithiumdihydrogencitrat, Dilithiumhydrogencitrat, Trilithiumcitrat, Ammoniumdihydrogencitrat, Diammoniumhydrogencitrat, Triammoniumcitrat, Tricalciumdicitrat (Calciumcitrat), Trimagnesiumdicitrat (Magnesiumcitrat) und/oder partielle Citratester enthält.

Nach der vorliegenden Erfindung ist es ebenso möglich, dass die Citrat-Lösung eine Mischung mehrerer der oben aufgeführten Citrat-Verbindungen enthält.

Der Begriff **"Citrat-Verbindung",** wie hierin verwendet, umfasst sowohl Zitronensäure als auch deren Salze.

Wird in dieser Anmeldung der recht allgemeine Begriff "Natriumcitrat" verwendet, umfasst dieser Begriff die verschieden protonierten Formen des Natriumcitrates, also sowohl die unprotonierte (Trinatriumcitrat), als auch die einfach protonierte (Dinatriumhydrogencitrat) oder die zweifach protonierte Form (Natriumdihydrogencitrat).

Wird in dieser Anmeldung der recht allgemeine Begriff "Kaliumcitrat" verwendet, umfasst dieser Begriff die verschieden protonierten Formen des Kaliumcitrates, also sowohl die unprotonierte (Trikaliumcitrat), als auch die einfach protonierte (Dikaliumhydrogencitrat) oder die zweifach protonierte Form (Kaliumdihydrogencitrat).

Wird in dieser Anmeldung der recht allgemeine Begriff "Lithiumcitrat" verwendet, umfasst dieser Begriff die verschieden protonierten Formen des Lithiumcitrates, also sowohl die unprotonierte (Trilithiumcitrat), als auch die einfach protonierte (Dilithiumhydrogencitrat) oder die zweifach protonierte Form (Lithiumdihydrogencitrat).

Wird in dieser Anmeldung der recht allgemeine Begriff "Ammoniumcitrat" verwendet, umfasst dieser Begriff die verschieden protonierten Formen des Ammoniumcitrates, also sowohl die unprotonierte (Triammoniumcitrat), als auch die einfach protonierte (Diammoniumhydrogencitrat) oder die zweifach protonierte Form (Ammoniumdihydrogencitrat).

Der Begriff **"partieller Citratester",** wie hierin verwendet, bezeichnet Ester der Zitronensäure, bei denen jedoch nicht alle drei Carboxyl-Gruppen (d.h. $-COO^-$) des Citrates verestert sind (d.h. -COOR, wobei R ein organsicher Rest ist), sondern höchstens zwei der drei Carboxyl-Gruppen des Citrates, bevorzugt jedoch höchstens eine der drei Carboxyl-Gruppen des Citrates verestert sind. Neben den ein bis zwei Estergruppen, enthält der partielle Citratester noch ein bis zwei physiologisch verträgliche anorganische Kationen (z.B. Metallkationen wie $Na^+$, $K^+$), welche die ein oder zwei nicht-veresterte(n) Carboxylgruppe(n) des Citrates komplexieren.

Eine bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,

wobei die Citrat-Lösung mindestens eine der Citrat-Verbindungen aus der Gruppe umfassend oder bestehend aus Zitronensäure, Natriumdihydrogencitrat, Dinatriumhydrogencitrat, Trinatriumcitrat, Trinatriumcitrat Dihydrat, Kaliumdihydrogencitrat, Dikaliumhydrogencitrat, Trikaliumcitrat, Lithiumdihydrogencitrat, Dilithiumhydrogencitrat, Trilithiumcitrat, Ammoniumdihydrogencitrat, Diammoniumhydrogencitrat, Triammoniumcitrat, Tricalciumdicitrat (Calciumcitrat), Trimagnesiumdicitrat (Magnesiumcitrat) und/oder partielle Citratester enthält,

wobei die mindestens eine Citrat-Verbindung ausgewählt wird aus Zitronensäure sowie Citrat-Salzen mit monovalenten Metallkationen.

Es wird erfindungsgemäß bevorzugt, wenn die Citrat-Lösung kein Eisen(II)citrat enthält. Noch mehr bevorzugt wird, wenn die Citrat-Lösung kein Eisen(III)citrat enthält. Zudem wird erfindungsgemäß bevorzugt, wenn die Citrat-Lösung kein Kupfer(II)citrat (Trikupferdicitrat) und kein Aluminiumcitrat enthält.

In weiteren bevorzugten Ausführungsformen der vorliegenden Erfindung enthält die Citrat-Lösung kein Calciumcitrat (auch als Tricalciumdicitrat bekannt).

In einer weiteren bevorzugten Ausführungsform der vorliegenden Erfindung enthält die Citrat-Lösung kein Magnesiumcitrat (auch als Trimagnesiumdicitrat bekannt) oder Magnesiumcitrat in einer Konzentration von höchstens 1 mM.

In einer weiteren bevorzugten Ausführungsform der vorliegenden Erfindung enthält die Citrat-Lösung **kein Calciumcitrat** und **kein Magnesiumcitrat** oder Magnesiumcitrat in einer Konzentration von höchstens 1 mM.

Wird in der vorliegenden Anmeldung erwähnt, dass eine Lösung eine bestimmte Substanz <u>nicht</u> enthält, bedeutet dies, dass diese Lösung die Substanz entweder überhaupt nicht enthält oder zumindest, um eventuellen unvermeidbaren Verunreinigungen Rechnung zu tragen, nicht über 0,01 Gewichtsprozent dieser Substanz enthält.

Eine besonders bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, wobei die Citrat-Lösung mindestens eine der Citrat-Verbindungen aus der Gruppe umfassend oder bestehend aus Zitronensäure, Natriumdihydrogencitrat, Dinatriumhydrogencitrat, Trinatriumcitrat, Trinatriumcitrat Dihydrat, Kaliumdihydrogencitrat, Dikaliumhydrogencitrat, Trikaliumcitrat, Lithiumdihydrogencitrat, Dilithiumhydrogencitrat, und/oder Trilithiumcitrat enthält. Noch bevorzugter werden jedoch Zitronensäure, Natriumdihydrogencitrat, Dinatriumhydrogencitrat, Trinatriumcitrat, Trinatriumcitrat Dihydrat, Kaliumdihydrogencitrat, Dikaliumhydrogencitrat und/oder Trikaliumcitrat. Am meisten bevorzugt werden jedoch Zitronensäure, Natriumdihydrogencitrat, Dinatriumhydrogencitrat, Trinatriumcitrat und Trinatriumcitrat Dihydrat.

Erfindungsgemäß ist weiterhin bevorzugt, wenn die Citrat-Lösung neben der besagten Citrat-Verbindung bzw. den besagten Citrat-Verbindungen keine zusätzlichen, das heißt weitere nicht-Citrat-basierte Substanzen, enthält, die $Ca^{2+}$ chelieren (so genannten $Ca^{2+}$-Chelatoren).

Eine bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, wobei die Citrat-Lösung keine $Ca^{2+}$-Chelatoren enthält, die chemisch nicht auf Citrat-Verbindungen basieren. Mögliche Beispiele für $Ca^{2+}$-Chelatoren, die chemisch nicht auf Citrat-Verbindungen basieren und somit nicht bevorzugt sind, sind EDTA (Ethylendiamintetraessigsäure), EGTA (Ethylenglycol-bis(aminoethylether)-N,N,N',N'-tetraessigsäure), BAPTA (1,2-Bis(o-aminophenoxy)ethan-N,N,N',N'-tetraessigsäure) sowie Oxalate.

Eine bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung mindestens eine der Citrat-Verbindungen aus der Gruppe umfassend oder bestehend aus Zitronensäure, Natriumdihydrogencitrat, Dinatriumhydrogencitrat, Trinatriumcitrat, Trinatriumcitrat Dihydrat, Kaliumdihydrogencitrat, Dikaliumhydrogencitrat, Trikaliumcitrat, Lithiumdihydrogencitrat, Dilithiumhydrogencitrat, Trilithiumcitrat, Ammoniumdihydrogencitrat, Diammoniumhydrogencitrat, Triammoniumcitrat, Tricalciumdicitrat (Calciumcitrat), Trimagnesiumdicitrat (Magnesiumcitrat) und/oder partielle Citratester enthält, wobei die Citrat-Lösung neben der/den Citrat-Verbindung(en) keine zusätzlichen $Ca^{2+}$-Chelatoren enthält.

Erfindungsgemäß ist es daher weiterhin bevorzugt, wenn die Citrat-Lösung kein EDTA (Ethylendiamintetraessigsäure), EGTA (Ethylenglycol-bis(aminoethylether)-N,N,N',N'-tetraessigsäure), BAPTA (1,2-Bis(o-aminophenoxy)ethan-N,N,N',N'-tetraessigsäure) oder Oxalat enthält.

Neben den oben angegebenen Citrat-Verbindungen kann die Citrat-Lösung zusätzliche Substanzen enthalten, die jedoch keine $Ca^{2+}$-chelierenden Eigenschaften aufweisen. So sind zum Beispiel weitere Substanzen als Zusätze möglich, die dazu beitragen, dass die Osmolarität der Citrat-Lösung in etwa der Osmolarität der biologischen Flüssigkeit, also z.B. des Blutes oder Blutplasmas, entspricht. Derartige Substanzen zur Einstellung der Osmolarität umfassen anorganische Salze wie z.B. Natriumchlorid, Kaliumchlorid aber auch Zucker wie z.B. Glucose, Dextrose (D-Glucose), Fructose und Saccharose oder Heparin. Die Begriffe Dextrose und D-Glucose sind gleichbedeutend und können hierin daher synonym verwendet werden.

Ebenso sind weitere Substanzen als Zusätze möglich, die dazu dienen, den pH-Wert der Citrat-Lösung auf einen vorgegebenen Wert, z.B. den pH-Wert der biologischen Flüssigkeit (also z.B. des Blutes oder Blutplasmas), einzustellen. Hier ist es z.B. möglich, dass die zusätzlich enthaltene Substanz zusammen mit der Citrat-Verbindung ein Puffersystem bildet, über das der pH-Wert der Citrat-Lösung eingestellt werden kann und leichte Schwankungen im pH-Wert kompensiert werden können. Enthält die Citrat-Lösung ein Puffer-System aus Citrat-Verbindung und einer Nicht-Citrat-Puffersubstanz, so wird auch der Begriff **"citrathaltiger Puffer"** verwendet. Mögliche Beispiele für zugesetzte Puffer-Substanzen, die keine Citrat-Verbindungen sind (also Nicht-Citrat-Puffersubstanzen), umfassen Dinatriumhydrogenphosphat, Natriumdihydrogenphosphat, Dikaliumhydrogenphosphat, Kaliumdihydrogenphosphat, Lactat und Acetat.

Mögliche Beispiele für Puffersysteme aus einer Citrat-Verbindung und einer nicht-Citrat-Puffersubstanz, also eine erfindungsgemäße Citrat-Lösung in Form eines citrathaltigen Puffers, ist die Kombination von Zitronensäure mit Dinatriumhydrogenphosphat, Natriumdihydrogenphosphat, Dikaliumhydrogenphosphat oder Kaliumdihydrogenphosphat. Besonders bevorzugt ist jedoch die Kombination von Zitronensäure mit Dinatriumhydrogenphosphat.

Enthalten jedoch die Citrat-Lösungen zwei unterschiedliche Citrat-Verbindungen, die gemeinsam ebenfalls ein Puffersystem darstellen, so wird auch der Begriff **"Citrat-Puffer"** verwendet.

Mögliche Beispiele für Puffersysteme aus zwei unterschiedlichen Citrat-Verbindungen, also eine erfindungsgemäße Citrat-Lösung in Form eines Citrat-Puffers, ist die Kombination von Zitronensäure mit Natriumdihydrogencitrat, Dinatriumhydrogencitrat, Trinatriumcitrat, Kaliumdihydrogencitrat, Dikaliumhydrogencitrat oder Trikaliumcitrat;

die Kombination von Natriumdihydrogencitrat mit Dinatriumhydrogencitrat, Trinatriumcitrat, Dikaliumhydrogencitrat oder Trikaliumcitrat;
die Kombination von Kaliumdihydrogencitrat mit Dinatriumhydrogencitrat, Trinatriumcitrat Dikaliumhydrogencitrat oder Trikaliumcitrat;
die Kombination von Dinatriumhydrogencitrat mit Trinatriumcitrat oder Trikaliumcitrat; sowie die Kombination von Dikaliumhydrogencitrat mit Trinatriumcitrat oder Trikaliumcitrat.

Besonders bevorzugt ist jedoch die Kombination von Zitronensäure mit Trinatriumcitrat als Citrat-Lösung in Form eines Citrat-Puffers.

Es ist natürlich ebenso denkbar, zu der Citrat-Lösung enthaltend zwei unterschiedliche Citrat-Verbindungen (also ein Citrat-Puffer) weiterhin Substanzen zur Einstellung der Osmolarität zuzugeben.

Erfindungsgemäß wird bevorzugt, wenn die Citrat-Lösung lediglich aus Wasser, einer oder mehreren der oben aufgeführten Citrat-Verbindungen, sowie optional aus einer oder mehreren der oben aufgeführten Verbindungen zur Einstellung des Osmolarität (auch als "Osmolytika" bezeichnet) und/oder optional aus einer oder mehreren der oben aufgeführten Nicht-Citrat-Puffersubstanzen besteht.

Eine weitere Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung aus Wasser; und
mindestens einer der Citrat-Verbindungen aus der Gruppe umfassend oder bestehend aus Zitronensäure, Natriumdihydrogencitrat, Dinatriumhydrogencitrat, Trinatriumcitrat, Trinatriumcitrat Dihydrat, Kaliumdihydrogencitrat, Dikaliumhydrogencitrat, Trikaliumcitrat, Lithiumdihydrogencitrat, Dilithiumhydrogencitrat, Trilithiumcitrat, Ammoniumdihydrogencitrat, Diammoniumhydrogencitrat, Triammoniumcitrat, Tricalciumdicitrat (Calciumcitrat), Trimagnesiumdicitrat (Magnesiumcitrat) und/oder partielle Citratester; und
(optional) mindestens einer der Verbindungen aus der Gruppe umfassend oder bestehend aus Natriumchlorid, Kaliumchlorid, Glucose, Fructose und Saccharose, und/oder
(optional) mindestens einer der Verbindungen aus der Gruppe umfassend oder bestehend aus Dinatriumhydrogenphosphat, Natriumdihydrogenphosphat, Dikaliumhydrogenphosphat, Kaliumdihydrogenphosphat, Lactat und Acetat besteht.

Eine bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung aus Zitronensäure, Trinatriumcitrat und Wasser besteht.

Eine besonders bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung 38 mM Zitronensäure, 74,8 mM Trinatriumcitrat, und Wasser enthält.

Eine bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung aus Zitronensäure, Trinatriumcitrat, D-Glucose und Wasser besteht.

Eine Citrat-Lösung bestehend aus Zitronensäure, Trinatriumcitrat, D-Glucose und Wasser wird auch als **"Acid-Citrat-Dextrose-Lösung (ACD-Lösung)"** bezeichnet.

Bevorzugte Varianten der erfindungsgemäß verwendeten Citrat-Lösung betreffen ACD-Lösungen, welche zwischen 22,9 mM und 38,0 mM Zitronensäure, zwischen 44,9 mM und 74,8 mM Trinatriumcitrat, zwischen 74,2 mM und 123,6 mM D-Glucose und Wasser enthalten.

Eine besonders bevorzugte Variante der erfindungsgemäß verwendeten Citrat-Lösung betrifft eine ACD-Lösung, welche 38 mM Zitronensäure, 74,8 mM Trinatriumcitrat, 123,6 mM D-Glucose und Wasser enthält. Diese wird auch als "ACD-A Lösung" bezeichnet.

Eine besonders bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung 38 mM Zitronensäure, 74,8 mM Trinatriumcitrat, 123,6 mM D-Glucose und Wasser enthält.

Eine weitere bevorzugte Ausführungsform der vorliegenden Erfindung betrifft die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung aus Zitronensäure, Trinatriumcitrat, Natriumhydrogenphosphat, D-Glucose und Wasser besteht.

Eine Citrat-Lösung bestehend aus Zitronensäure, Trinatriumcitrat, Natriumhydrogenphosphat, D-Glucose und Wasser wird auch als **"Citrat-Phosphat-Dextrose-Lösung (CPD)"** bezeichnet.

Eine bevorzugte Variante der erfindungsgemäß verwendeten Citrat-Lösung betrifft eine CPD-Lösung, welche 15,6 mM Zitronensäure, 89,4 mM Trinatriumcitrat, 128,7 mM D-Glucose, 16,1 mM Natriumhydrogenphosphat und Wasser enthält.

Eine besonders bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung 15,6 mM Zitronensäure, 89,4 mM Trinatriumcitrat, 128,7 mM D-Glucose, 16,1 mM Natriumhydrogenphosphat und Wasser enthält.

Eine weitere bevorzugte Ausführungsform der vorliegenden Erfindung betrifft die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung aus Zitronensäure, Trinatriumcitrat, Natriumhydrogenphosphat, D-Glucose, Adenin und Wasser besteht.

Eine Citrat-Lösung bestehend aus Zitronensäure, Trinatriumcitrat, Natriumhydrogenphosphat, D-Glucose, Adenin und Wasser wird auch als **"Citrat-Phosphat-Dextrose-Lösung mit Adenin (CPDA)"** bezeichnet.

Eine bevorzugte Variante der erfindungsgemäß verwendeten Citrat-Lösung betrifft eine CPDA-Lösung, welche 15,6 mM Zitronensäure, 89,4 mM Trinatriumcitrat, zwischen 128,7 mM und 160,9 mM D-Glucose, 16,1 mM Natriumhydrogenphosphat, 2 mM Adenin und Wasser enthält.

Eine bevorzugte Variante der erfindungsgemäß verwendeten Citrat-Lösung betrifft eine CPDA-Lösung, welche 15,6 mM Zitronensäure, 89,4 mM Trinatriumcitrat, zwischen 128,7 mM D-Glucose, 16,1 mM Natriumhydrogenphosphat, 2 mM Adenin und Wasser enthält.

Eine besonders bevorzugte Variante der erfindungsgemäß verwendeten Citrat-Lösung betrifft eine CPDA-Lösung, welche 15,6 mM Zitronensäure, 89,4 mM Trinatriumcitrat, 160,9 mM D-Glucose, 16,1 mM Natriumhydrogenphosphat, 2 mM Adenin und Wasser enthält.

Eine besonders bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung 15,6 mM Zitronensäure, 89,4 mM Trinatriumcitrat, zwischen 128,7 mM und 160,9 mM D-Glucose, 16,1 mM Natriumhydrogenphosphat, 2 mM Adenin und Wasser enthält.

Gemäß einigen Ausführungsformen der vorliegenden Erfindung wird bevorzugt, wenn die Citrat-Lösung keine Metallsalze mit mehrwertigen, d.h. zweiwertigen oder dreiwertigen, Kationen enthält. Daher enthält in einer weiteren bevorzugten Ausführungsform der vorliegenden Erfindung die Citrat-Lösung kein Calciumsalz. In einer weiteren bevorzugten Ausführungsform der vorliegenden Erfindung enthält die Citrat-Lösung kein Magnesiumsalz oder zumindest ein Magnesiumsalz in einer Konzentration von höchstens 1 mM.

Anders ausgedrückt wird gemäß einigen Ausführungsformen der vorliegenden Erfindung bevorzugt, wenn die Citrat-Lösung **nur Metallsalze mit einwertigen Kationen** enthält.

## Citrat-Konzentration und Verdünnung mit biologischer Flüssigkeit

Eine bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung eine Gesamtkonzentration an Citrat in einem Bereich von 50 mM bis 200 mM, bevorzugt von 60 mM bis 150 mM, noch bevorzugter 70 mM bis 120 mM, noch bevorzugter 75 mM bis 115 mM, und am meisten bevorzugt von 74,8 mM bis 113 mM aufweist.

Eine bevorzugte Ausführungsform der vorliegenden Erfindung betrifft daher ebenso die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,

wobei die Citrat-Lösung eine Gesamtkonzentration an Citrat von vorzugsweise 50 mM, von 150 mM, von 200 mM, bevorzugter von 60 mM, bevorzugter von 120 mM, noch bevorzugter von 115 mM, noch bevorzugter von 70 mM, noch bevorzugter von 75 mM, noch bevorzugter von 74,8 mM, am meisten bevorzugt von 113mM aufweist.

Natürlich ist nicht nur die Konzentration an Citrat in der eigentlichen Citrat-Lösung von Bedeutung, sondern vor allem die letztliche Konzentration in dem Gemisch aus Citrat-Lösung und biologischer Flüssigkeit, also z.B. Blut oder Blutplasma. Daher ist auch die verwendete Verdünnung der Citrat-Lösung von Bedeutung.

Daher betrifft die vorliegende Erfindung auch die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, wobei die biologische Flüssigkeit vor der Entfernung von CRP mit der Citrat-Lösung vermischt wird.

Ebenso betrifft die vorliegende Erfindung die Verwendung einer Citrat-Lösung als Bindungspuffer (oder Bindungslösung) zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, wobei die biologische Flüssigkeit vor der Entfernung von CRP mit der Citrat-Lösung vermischt wird.

Die vorliegende Erfindung betrifft daher auch die Verwendung einer Citrat-Lösung zur affinitätschromatographischen Entfernung von CRP aus biologischen Flüssigkeiten, wobei die affinitätschromatographische Entfernung von CRP durch (eine $Ca^{2+}$-abhängige) Bindung von CRP an ein mit ω-Phosphonooxyalkylammoniumgruppen und/oder mit ω-Ammoniumalkoxy-hydroxy-phosphoryloxygruppen funktionalisiertes Säulenmaterial erfolgt, und wobei die biologische Flüssigkeit vor der Entfernung von CRP mit der Citrat-Lösung vermischt wird.

Im Anschluss an die Vermischung der Citrat-Lösung mit der biologischen Flüssigkeit (also z.B. Blut oder Blutplasma), wird das Gemisch aus Citrat-Lösung und biologischer Flüssigkeit auf das Säulenmaterial, welches mit ω-Phosphonooxyalkylammoniumgruppen und/oder mit ω-Ammoniumalkoxy-hydroxy-phosphoryloxygruppen funktionalisiert wurde, aufgetragen, so dass eine ($Ca^{2+}$-abhängige) Bindung von CRP an das funktionalisierte Säulenmaterial stattfindet.

Die vorliegende Erfindung betrifft daher ebenso die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, wobei die Citrat-Lösung in einem Bereich von 1:50 bis 1:5, vorzugsweise von 1:40 bis 1:10, vorzugsweise von 1:30 bis 1:20, vorzugsweise von 1:20 bis 1:15, vorzugsweise von 1:15 bis 1:10 mit der biologischen Flüssigkeit verdünnt wird.

Die vorliegende Erfindung betrifft daher ebenso die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, wobei die Citrat-Lösung 1:50, bevorzugt 1:45, weiter bevorzugt 1:40, bevorzugt 1:35, bevorzugt 1:30, bevorzugt 1:25, weiter bevorzugt 1:20, weiter bevorzugt 1:15, weiter bevorzugt 1:10, bevorzugt 1:5 mit der biologischen Flüssigkeit verdünnt wird.

Eine bevorzugte Ausführungsform der vorliegenden Erfindung betrifft daher die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
in einer Verdünnung in einem Bereich von 1:50 bis 1:5 mit der biologischen Flüssigkeit.

Eine bevorzugte Ausführungsform der vorliegenden Erfindung betrifft daher auch die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung eine Gesamtkonzentration an Citrat in einem Bereich von 50 mM bis 200 mM, bevorzugt von 60 mM bis 150 mM, noch bevorzugter 70 mM bis 120 mM, noch bevorzugter 75 mM bis 115 mM, und am meisten bevorzugt von 74,8 mM bis 113 mM; und
wobei die Citrat-Lösung in einem Bereich von 1:50 bis 1:5, vorzugsweise von 1:40 bis 1:10, vorzugsweise von 1:30 bis 1:20, vorzugsweise von 1:20 bis 1:15, vorzugsweise von 1:15 bis 1:10 mit der biologischen Flüssigkeit verdünnt wird.

Eine weiter bevorzugte Ausführungsform der vorliegenden Erfindung betrifft daher auch die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung eine Gesamtkonzentration an Citrat in einem Bereich von 70 mM bis 120 mM; und
wobei die Citrat-Lösung in einem Bereich von 1:50 bis 1:5 mit der biologischen Flüssigkeit verdünnt wird.

Es ist natürlich auch denkbar, dass Citrat-Lösungen verwendet werden, die eine geringere oder auch höhere Citrat-Konzentration enthalten als in den vorgehenden Absätzen beschrieben. Dementsprechend müsste natürlich der

Verdünnungsgrad mit der biologischen Flüssigkeit entsprechend verringert (bei niedrigeren Citrat-Konzentrationen) oder erhöht (bei höheren Citrat-Konzentrationen) werden. Die in den vorhergehenden Absätzen beschriebenen Citrat-Konzentrationen und Verdünnungen haben sich jedoch als besonders vorteilhaft im Zusammenhang mit der erfindungsgemäßen Verwendung einer Citrat-Lösung zur affinitätschromatographischen Entfernung von CRP aus biologischen Flüssigkeiten erwiesen.

Da der pH-Wert einer Lösung einen wesentlichen Einfluss auf die Dissoziation von Säuren und Basen (also auch z.B. von Zitronensäure) hat, und somit im vorliegenden Fall auch die Bindung von $Ca^{2+}$ beeinflusst, ist der pH-Wert der verwendeten Citrat-Lösung ein wichtiger Aspekt der vorliegenden Erfindung.

Die vorliegende Erfindung betrifft daher auch die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials,
wobei die Citrat-Lösung einen pH-Wert in einem Bereich von pH 4,0 bis pH 7,0 aufweist, bevorzugt von pH 4,5 bis pH 6,5, weiter bevorzugt von pH 4,7 bis pH 6,0, noch weiter bevorzugt von pH 4,9 bis pH 5,8, noch weiter bevorzugt von pH 5,0 bis pH 5,6, und am meisten bevorzugt von pH 5,0 bis pH 5,5.

## $Ca^{2+}$-abhängige Liganden für CRP

Wie bereits mehrfach erwähnt, wird für die affinitätschromatographische Entfernung von CRP aus biologischen Flüssigkeiten, z.B. aus Blut oder Blutplasma, ein Säulenmaterial verwendet, dass Phosphocholin und/oder Phosphoethanolamin bzw. deren Derivate enthält, wodurch eine $Ca^{2+}$-abhängige Bindung von CRP an das besagte funktionalisierte Säulenmaterial möglich ist.

Hierzu wird Phosphocholin, Phosphoethanolamin oder deren Derivate an einem Säulenmaterial immobilisiert. Dies geschieht in der Regel über eine organische Linker-Gruppe, über die das Phosphocholin, Phosphoethanolamin bzw. deren Derivate adsorptiv oder noch bevorzugter kovalent mit dem Säulenmaterial verbunden ist. Die resultiert in einem so genannten **"funktionalisierten Säulenmaterial",** wobei die für die $Ca^{2+}$-abhängige Bindung von CRP verantwortliche chemische Gruppe nach außen exponiert ist, so dass CRP, das sich in einer biologischen Flüssigkeit befindet, auch Zugang zu der besagten chemischen Gruppe hat.

Anders ausgedrückt, bezeichnet der Begriff **"funktionalisiertes Säulenmaterial",** wie hierin verwendet, ein Säulenmaterial zur Affinitätschromatographie, welches mit einer funktionalen chemischen Gruppe versehen wurde. Hierbei kann die funktionale chemische Gruppe über adsorptive oder ionische Wechselwirkungen aber bevorzugt über eine kovalente Bindung mit dem Säulenmaterial verbunden sein. Es ist natürlich von Bedeutung, dass die funktionale chemische Gruppe derart mit dem Säulenmaterial verbunden ist, dass die funktionale Gruppe aktiv und exponiert ist, so dass ihre Funktionalität erhalten bleibt. Hierdurch ist es möglich, dass die am Säulenmaterial befestigte Gruppe (hier: ω-Phosphonooxyalkylammoniumgruppe und/oder ω-Ammoniumalkoxy-hydroxy-phosphoryloxygruppe) mit einem Liganden (hier: CRP) aus der Probe (hier: biologische Flüssigkeit wie z.B. Blut oder Blutplasma) interagieren kann bzw. diesen binden kann.

Je nachdem, ob das Phosphocholin, Phosphoethanolamin bzw. deren Derivat über die Ammoniumgruppe oder über die Phosphatgruppe über einen organischen Linker mit dem Säulenmaterial verbunden ist, so unterscheidet man zwischen einem Säulenmaterial, das mit einer ω-Phosphonooxyalkylammoniumgruppe funktionalisiert wurde (Verknüpfung über die Ammoniumgruppe), und einem Säulenmaterial, das mit einer ω-Ammoniumalkoxy-hydroxy-phosphoryloxygruppe funktionalisiert wurde (Verknüpfung über die Phosphatgruppe).

Die Verknüpfung mit dem Säulenmaterial (gegebenenfalls über einen organischen Linker) wird in den unten aufgeführten Formeln (**I**) und (**II**) über eine **gestrichelte Linie** entweder am Stickstoffatom der Ammoniumgruppe oder am Sauerstoffatom der Phosphatgruppe dargestellt.

Der Begriff **"ω-Phosphonooxyalkylammoniumgruppe"** wie hierin verwendet, kann gleichbedeutend mit "omega-Phosphonooxyalkylammonium" verwendet werden und beschreibt Verbindungen der folgenden allgemeinen Formel (**I**)

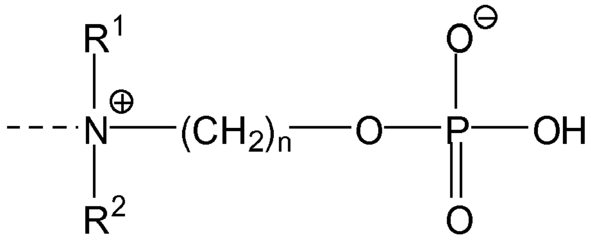

(**I**)

wobei
n ausgewählt wird aus 2 und 3;
$R^1$ und $R^2$ unabhängig voneinander ausgewählt werden aus: -H, $-CH_3$, $-C_2H_5$, $-C_3H_7$, $-C_4H_9$, $-C_5H_{11}$, $-C_6H_{13}$, oder $R^1$ und $R^2$ zusammen mit dem Stickstoffatom mit dem sie verbunden sind einen Heterozyklus bilden können, der ausgewählt wird aus:

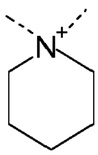 , 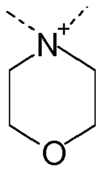 , 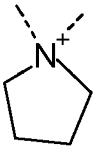 ,

wobei ein oder mehrere Wasserstoffatom(e) durch (ein) Fluoratom(e) ersetzt werden können.

Die vorliegende Erfindung ist daher ebenso gerichtet auf die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, dadurch gekennzeichnet, dass die ω-Phosphonooxyalkylammoniumgruppen die folgende allgemeine Formel (**I**) aufweisen

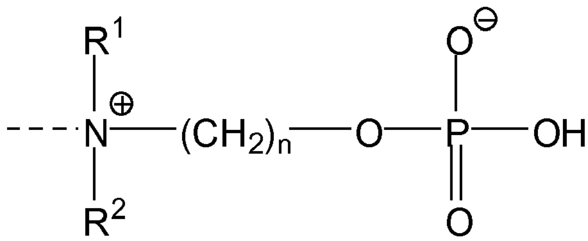

(**I**)

wobei
n ausgewählt wird aus 2 und 3;
$R^1$ und $R^2$ unabhängig voneinander ausgewählt werden aus: -H, $-CH_3$, $-C_2H_5$, $-C_3H_7$, $-C_4H_9$, $-C_5H_{11}$, $-C_6H_{13}$, oder $R^1$ und $R^2$ zusammen mit dem Stickstoffatom mit dem sie verbunden sind einen Heterozyklus bilden können, der ausgewählt wird aus:

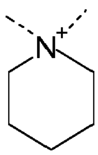 , 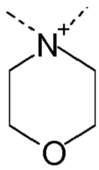 , 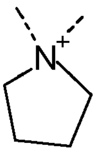 ,

wobei ein oder mehrere Wasserstoffatom(e) durch (ein) Fluoratom(e) ersetzt werden können.

Bevorzugte ω-Phosphonooxyalkylammoniumgruppen umfassen Verbindungen der allgemeinen Formel (**I**)

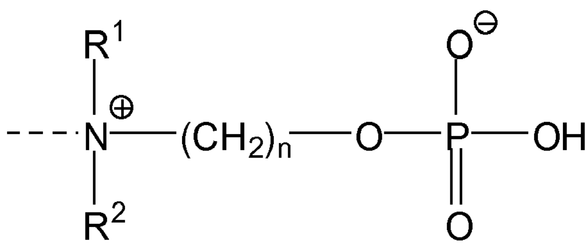

(**I**)

wobei
n = 2 oder 3 ist;
$R^1$ und $R^2$ unabhängig voneinander ausgewählt werden aus: -H, $-CH_3$, $-C_2H_5$,
$-C_3H_7$ oder $R^1$ und $R^2$ zusammen mit dem Stickstoffatom mit dem sie verbunden sind einen Heterozyklus bilden können, der ausgewählt wird aus:

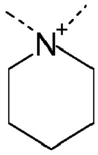
,
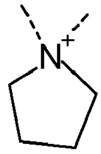
.

Besonders bevorzugte ω-Phosphonooxyalkylammoniumgruppen umfassen Verbindungen der allgemeinen Formel (**I**)

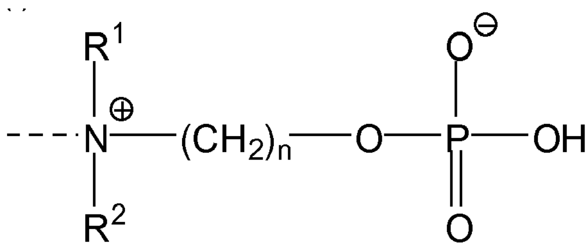

(**I**)

wobei
n = 2 ist;
$R^1$ und $R^2$ ausgewählt werden aus: -H, $-CH_3$, $-C_2H_5$, und besonders bevorzugt aus $-CH_3$ und $-C_2H_5$ oder $R^1$ und $R^2$ zusammen mit dem Stickstoffatom mit dem sie verbunden sind einen Heterozyklus bilden können, der ausgewählt wird aus:

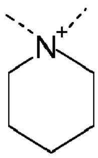
,
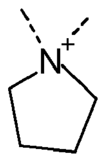
.

Bevorzugte Verbindungen, die eine wie oben beschriebene ω-Phosphonooxyalkylammoniumgruppe enthalten und für die Funktionalsierung eines entsprechendes Säulenmaterials geeignet sind umfassen beispielsweise: 2-[2-(2-Aminoethoxy)ethyl-diethyl-ammonio]ethyl-hydrogenphosphat, 2-[4-[2-(2-Aminoethoxy)ethyl]morpholin-4-ium-4-yl]ethyl-hydrogenphosphat, 2-[1-[2-(2-Aminoethoxy)ethyl]piperidin-1-ium-1-yl]ethyl-hydrogenphosphat, 2-[2-(2-Aminoethoxy)ethyl-dimethyl-ammonio]ethyl-hydrogenphosphat, 2-[3-Aminopropyl(dimethyl)ammonio]ethyl-hydrogenphosphat, 2-[Dimethyl(4-sulfanylbutyl)ammonio]ethyl-hydrogenphosphat, 2-[4-Azidobutyl(dimethyl)ammonio]ethyl-hydrogenphosphat, 2-[Dimethyl(pent-4-ynyl)ammonio]ethyl-hydrogenphosphat, 2-[3-(6-Aminohexanoylamino)propyl-diethyl-ammonio]ethyl-hydrogenphosphat, 2-[1-[2-[2-(6-Aminohexanoylamino)ethoxy]ethyl]piperidin-1-ium-1-yl]ethyl-hydrogenphosphat, 2-[4-[2-[2-[3-(6-Aminohexanoylamino)propanoylamino]ethoxy]ethyl]morpholin-4-ium-4-yl]ethyl-hydrogenphosphat, 2-[1-[2-[2-[6-(6-Aminohexanoylamino)hexanoylamino]ethoxy]-ethyl]pyrrolidin-1-ium-1-yl]ethyl-hydrogenphosphat, 2-[2-Allyloxyethyl(dimethyl)ammonio]ethyl-hydrogenphosphat, 2-[2-Allyloxyethyl(diethyl)ammonio]ethyl-hydrogenphosphat, 2-[4-(2-Allyloxyethyl)morpholin-4-ium-4-yl]ethyl-hydrogenphosphat, 2-[1-(2-Allyloxyethyl)piperidin-1-ium-1-yl]ethyl-hydrogenphosphat, 2-[2-[2-(6-Aminohexanoylamino)ethoxy]ethyl-dimethyl-ammonio]ethyl-hydrogenphosphat, 2-[2-[2-[3-(6-Aminohexanoylamino)propanoylamino]ethoxy]ethyl-dimethyl-ammonio]ethyl-hydrogenphosphat, 2-[3-Azidopropyl(dimethyl)ammonio]ethyl-hydrogenphosphat, 2-[Dimethyl-[2-[2-(prop-2-ynoxycarbonylamino)ethoxy]ethyl]ammonio]ethyl-hydrogenphosphat, 2-[2-[2-(Allyloxycarbonylamino)ethoxy]ethyl-dimethyl-ammonio]ethyl-hydrogenphosphat, 2-[2-[2-[6-(Allyloxycarbonylamino)hexanoylamino]ethoxy]ethyl-dimethyl-ammonio]ethyl-hydrogenphosphat, 2-[2-(6-Aminohexanoylamino)ethyl-dimethyl-ammonio]ethyl-hydrogenphosphat, 2-[Dimethyl-[3-[6-(prop-2-ynoxycarbonylamino)hexanoylamino]propyl]ammonio]ethyl-hydrogenphosphat, sowie 2-[3-(6-Aminohexanoylamino)propyl-dimethyl-ammonio]ethyl-hydrogenphosphat.

Der Begriff **"ω-Ammoniumalkoxy-hydroxy-phosphoryloxygruppen"** wie hierin verwendet, kann gleichbedeutend mit "omega-Ammoniumalkoxy-hydroxy-phosphoryloxygruppen" verwendet werden und beschreibt Verbindungen der folgenden allgemeinen Formel (**II**)

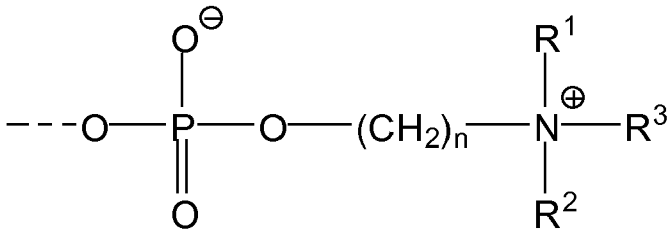

(**II**)

wobei
n ausgewählt wird aus 2 und 3;
$R^1$, $R^2$ und $R^3$ unabhängig voneinander ausgewählt werden aus: -H, $-CH_3$, $-C_2H_5$, $-C_3H_7$, $-C_4H_9$, $-C_5H_{11}$, $-C_6H_{13}$, oder $R^1$ und $R^2$ zusammen mit dem Stickstoffatom mit dem sie verbunden sind einen Heterozyklus bilden können, der ausgewählt wird aus:

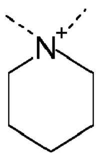 , 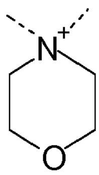 , 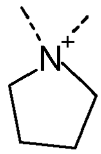 ,

und
$R^3$ ausgewählt wird aus: -H, $-CH_3$, $-C_2H_5$, $-C_3H_7$, $-C_4H_9$, $-C_5H_{11}$, $-C_6H_{13}$, und vorzugsweise -H;
wobei ein oder mehrere Wasserstoffatom(e) durch (ein) Fluoratom(e) ersetzt werden können.

Bevorzugte ω-Ammoniumalkoxy-hydroxy-phosphoryloxygruppen umfassen Verbindungen der allgemeinen Formel (**II**)

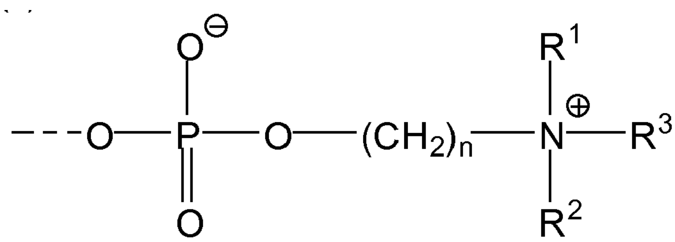

(**II**)

wobei
n ausgewählt wird aus 2 und 3;
$R^1$, $R^2$ und $R^3$ unabhängig voneinander ausgewählt werden aus: -H, $-CH_3$, $-C_2H_5$, $-C_3H_7$,
oder $R^1$ und $R^2$ zusammen mit dem Stickstoffatom mit dem sie verbunden sind einen Heterozyklus bilden können, der ausgewählt wird aus:

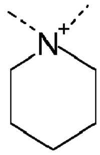 ,
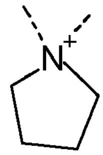 ,

und $R^3$ = -H ist.

Im Rahmen der vorliegenden Erfindung ist besonders bevorzugt, wenn es sich bei der ω-Ammoniumalkoxy-hydroxy-phosphoryloxygruppe um eine ω-Trialkylammoniumalkoxy-hydroxy-phosphoryloxygruppe handelt.

Daher umfassen besonders bevorzugte ω-Ammoniumalkoxy-hydroxy-phosphoryloxygruppen Verbindungen der allgemeinen Formel (**II**)

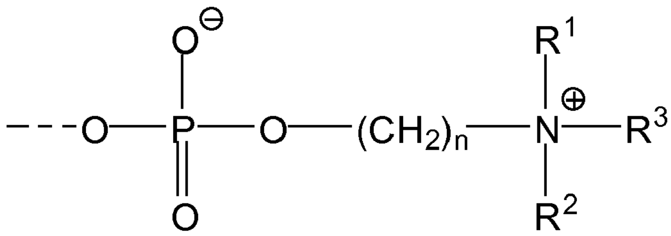

**(II)**

wobei
n = 2 ist;
und $R^1$, $R^2$ und $R^3$ ausgewählt werden aus: -H, $-CH_3$, $-C_2H_5$ und besonders bevorzugt aus $-CH_3$ und $-C_2H_5$.

Ebenso besonders bevorzugt ist daher, wenn es sich bei den ω-Ammoniumalkoxy-hydroxy-phosphoryloxygruppen um ω-Trimethylammoniumethoxy-hydroxy-phosphoryloxygruppen oder um ω-Trimethylammoniupropoxy-hydroxy-phosphoryloxygruppen handelt.

Bevorzugte Verbindungen, die eine wie oben beschriebene ω-Ammoniumalkoxy-hydroxy-phosphoryloxygruppe enthalten und für die Funktionalsierung eines entsprechendes Säulenmaterials geeignet sind, umfassen beispielsweise: p-Aminophenylphosphocholin (APPC), 4-[[Hydroxy[2-(trimethylammonio)ethoxy]phosphinyl]oxy]-benzoldiazonium (p-Diazonium-phenylphosphocholin) oder p-Nitrophenyl-6-(O-Phosphocholin)hydroxyhexanoat.

Die vorliegende Erfindung ist auch gerichtet auf die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, dadurch gekennzeichnet, dass die ω-Ammoniumalkoxy-hydroxy-phosphoryloxygruppen die folgende allgemeine Formel (**II**) aufweisen

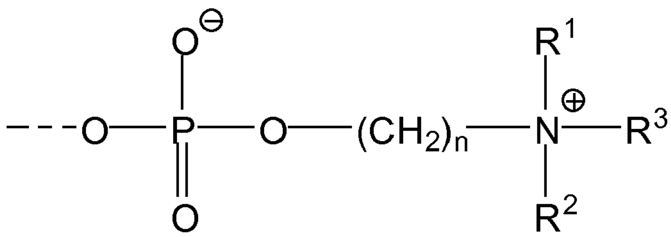

**(II)**

wobei
n ausgewählt wird aus 2 und 3;
$R^1$, $R^2$ und $R^3$ unabhängig voneinander ausgewählt werden aus: -H, $-CH_3$, $-C_2H_5$, $-C_3H_7$, $-C_4H_9$, $-C_5H_{11}$, $-C_6H_{13}$,
oder $R^1$ und $R^2$ zusammen mit dem Stickstoffatom mit dem sie verbunden sind einen Heterozyklus bilden können, der ausgewählt wird aus:

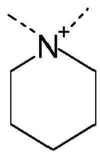,
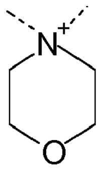,
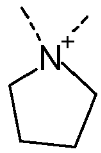,

und

$R^3$ ausgewählt wird aus: -H, $-CH_3$, $-C_2H_5$, $-C_3H_7$, $-C_4H_9$, $-C_5H_{11}$, $-C_6H_{13}$, und vorzugsweise -H;

wobei ein oder mehrere Wasserstoffatom(e) durch (ein) Fluoratom(e) ersetzt werden können.

Die vorliegende Erfindung ist daher ebenso gerichtet auf die Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials, dadurch gekennzeichnet, dass die ω-Phosphonooxyalkylammoniumgruppen die folgende allgemeine Formel (**I**) aufweisen

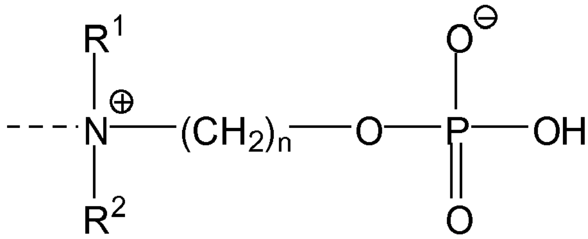

(**I**)

wobei

n ausgewählt wird aus 2 und 3;

$R^1$ und $R^2$ unabhängig voneinander ausgewählt werden aus: -H, $-CH_3$, $-C_2H_5$, $-C_3H_7$, $-C_4H_9$, $-C_5H_{11}$, $-C_6H_{13}$, oder $R^1$ und $R^2$ zusammen mit dem Stickstoffatom mit dem sie verbunden sind einen Heterozyklus bilden können, der ausgewählt wird aus:

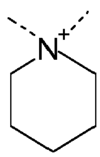,
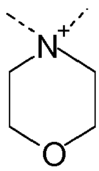,
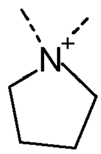,

wobei ein oder mehrere Wasserstoffatom(e) durch (ein) Fluoratom(e) ersetzt werden können;

und dadurch gekennzeichnet, dass die ω-Ammoniumalkoxy-hydroxy-phosphoryloxygruppen die folgende allgemeine Formel (**II**) aufweisen

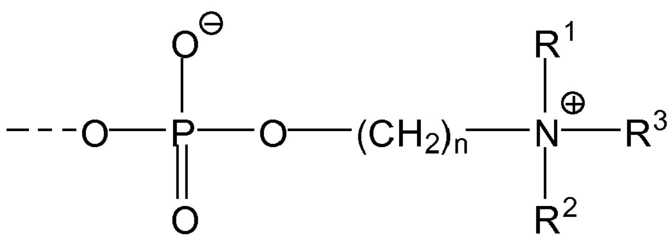

(**II**)

wobei

n ausgewählt wird aus 2 und 3;

$R^1$, $R^2$ und $R^3$ unabhängig voneinander ausgewählt werden aus: -H, $-CH_3$, $-C_2H_5$, $-C_3H_7$, $-C_4H_9$, $-C_5H_{11}$, $-C_6H_{13}$,

oder $R^1$ und $R^2$ zusammen mit dem Stickstoffatom mit dem sie verbunden sind einen Heterozyklus bilden können, der ausgewählt wird aus:

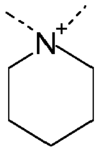,
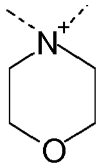,
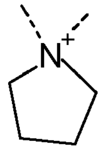,

und
$R^3$ ausgewählt wird aus: -H, $-CH_3$, $-C_2H_5$, $-C_3H_7$, $-C_4H_9$, $-C_5H_{11}$, $-C_6H_{13}$, und vorzugsweise -H;
wobei ein oder mehrere Wasserstoffatom(e) durch (ein) Fluoratom(e) ersetzt werden können.

Daher betrifft die vorliegende Erfindung ebenso die Verwendung einer Citrat-Lösung zur affinitätschromatographischen Entfernung von CRP aus biologischen Flüssigkeiten, wobei die affinitätschromatographische Entfernung von CRP durch (eine $Ca^{2+}$-abhängige) Bindung von CRP an ein mit ω-Phosphonooxyalkylammoniumgruppen und/oder mit ω-Ammoniumalkoxy-hydroxy-phosphoryloxygruppen funktionalisiertes Säulenmaterial erfolgt, wobei die ω-Phosphonooxyalkylammoniumgruppen die folgende allgemeine Formel (**I**) aufweisen

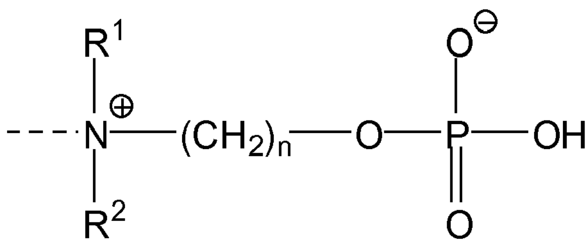

(**I**)

wobei
n ausgewählt wird aus 2 und 3;
$R^1$ und $R^2$ unabhängig voneinander ausgewählt werden aus: -H, $-CH_3$, $-C_2H_5$, $-C_3H_7$, $-C_4H_9$, $-C_5H_{11}$, $-C_6H_{13}$, oder $R^1$ und $R^2$ zusammen mit dem Stickstoffatom mit dem sie verbunden sind einen Heterozyklus bilden können, der ausgewählt wird aus:

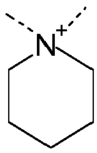,
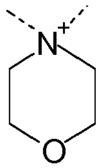,
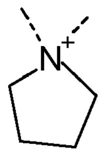,

wobei ein oder mehrere Wasserstoffatom(e) durch (ein) Fluoratom(e) ersetzt werden können.

Die vorliegende Erfindung ist auch gerichtet auf die Verwendung einer Citrat-Lösung zur affinitätschromatographischen Entfernung von CRP aus biologischen Flüssigkeiten, wobei die affinitätschromatographische Entfernung von CRP durch (eine $Ca^{2+}$-abhängige) Bindung von CRP an ein mit ω-Phosphonooxyalkylammoniumgruppen und/oder mit ω-Ammoniumalkoxy-hydroxy-phosphoryloxygruppen funktionalisiertes Säulenmaterial erfolgt, wobei die ω-Ammoniumalkoxy-hydroxy-phosphoryloxygruppen die folgende allgemeine Formel (**II**) aufweisen

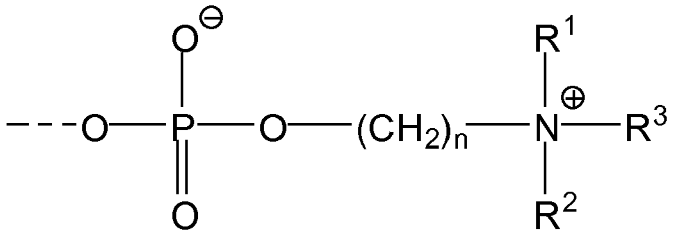

**(II)**

wobei
n ausgewählt wird aus 2 und 3;
$R^1$, $R^2$ und $R^3$ unabhängig voneinander ausgewählt werden aus: -H, $-CH_3$, $-C_2H_5$, $-C_3H_7$, $-C_4H_9$, $-C_5H_{11}$, $-C_6H_{13}$, oder $R^1$ und $R^2$ zusammen mit dem Stickstoffatom mit dem sie verbunden sind einen Heterozyklus bilden können, der ausgewählt wird aus:

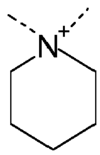 , 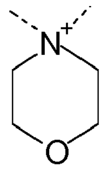 , 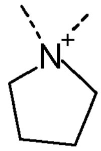 ,

und
$R^3$ ausgewählt wird aus: -H, $-CH_3$, $-C_2H_5$, $-C_3H_7$, $-C_4H_9$, $-C_5H_{11}$, $-C_6H_{13}$, und vorzugsweise -H;
wobei ein oder mehrere Wasserstoffatom(e) durch (ein) Fluoratom(e) ersetzt werden können.

Daher betrifft die vorliegende Erfindung ebenso die Verwendung einer Citrat-Lösung zur affinitätschromatographischen Entfernung von CRP aus biologischen Flüssigkeiten, wobei die affinitätschromatographische Entfernung von CRP durch (eine $Ca^{2+}$-abhängige) Bindung von CRP an ein mit ω-Phosphonooxyalkylammoniumgruppen und/oder mit ω-Ammoniumalkoxy-hydroxy-phosphoryloxygruppen funktionalisiertes Säulenmaterial erfolgt, wobei die ω-Phosphonooxyalkylammoniumgruppen die folgende allgemeine Formel (**I**) aufweisen

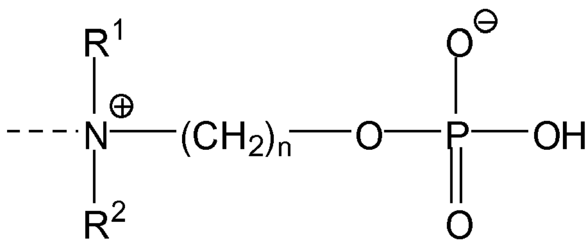

**(I)**

wobei
n ausgewählt wird aus 2 und 3;
$R^1$ und $R^2$ unabhängig voneinander ausgewählt werden aus: -H, $-CH_3$, $-C_2H_5$, $-C_3H_7$, $-C_4H_9$, $-C_5H_{11}$, $-C_6H_{13}$, oder $R^1$ und $R^2$ zusammen mit dem Stickstoffatom mit dem sie verbunden sind einen Heterozyklus bilden können, der ausgewählt wird aus:

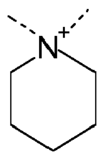 , 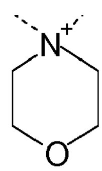 , 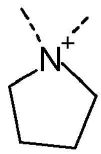 ,

wobei ein oder mehrere Wasserstoffatom(e) durch (ein) Fluoratom(e) ersetzt werden können;

und wobei die ω-Ammoniumalkoxy-hydroxy-phosphoryloxygruppen die folgende allgemeine Formel (**II**) aufweisen

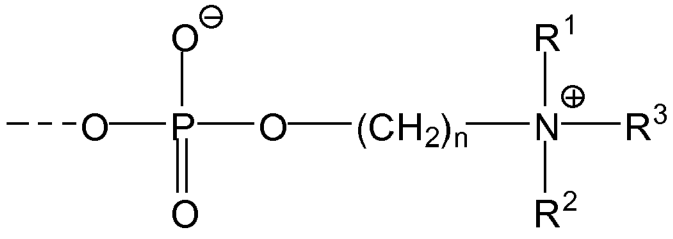

**(II)**

wobei
n ausgewählt wird aus 2 und 3;
$R^1$, $R^2$ und $R^3$ unabhängig voneinander ausgewählt werden aus: -H, $-CH_3$, $-C_2H_5$, $-C_3H_7$, $-C_4H_9$, $-C_5H_{11}$, $-C_6H_{13}$, oder $R^1$ und $R^2$ zusammen mit dem Stickstoffatom mit dem sie verbunden sind einen Heterozyklus bilden können, der ausgewählt wird aus:

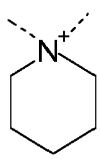 , 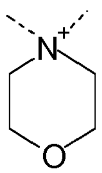 , 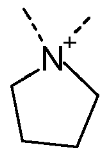 ,

und
$R^3$ ausgewählt wird aus: -H, $-CH_3$, $-C_2H_5$, $-C_3H_7$, $-C_4H_9$, $-C_5H_{11}$, $-C_6H_{13}$, und vorzugsweise -H;
wobei ein oder mehrere Wasserstoffatom(e) durch (ein) Fluoratom(e) ersetzt werden können.

In einer möglichen Ausführungsform der vorliegenden Erfindung wird die ω-Ammoniumalkoxy-hydroxy-phosphoryloxygruppe über eine Phosphoester-Bindung mit einer Hydroxygruppe eines Glycerin-Moleküls (als organsicher Linker) verknüpft, wobei der resultierende Glycerinester dann über eine zweite Hydroxygruppe des Glycerins mit dem Säulenmaterial verknüpft wird. Bei einer solchen Ausführungsform ist es zudem möglich, dass die verbleibende dritte Hydroxygruppe des Glycerins entweder mit einer Fettsäure verestert ist oder mit einer zweiten ω-Ammoniumalkoxy-hydroxy-phosphoryloxygruppe verestert ist. Zudem kann die Position der jeweiligen Veresterung am Glycerin-Molekül variiieren. Als Fettsäuren können die üblichen gesättigten, monoolefinischen, polyolefinischen, monoacetylenisch, ungesättigten linearen und/oder verzeigten Fettsäuren mit 8 bis 28 Kohlenstoffatomen dienen. Bevorzugte Fettsäurereste sind Palmitinsäure, Arachidonsäure, Oxovaleriansäure, Glutarsäure, Epoxyisoprostan und Stearinsäure.

## Säulenmaterial

Grundsätzlich sind für die Herstellung des mit ω-Phosphonooxyalkylammoniumgruppen und/oder mit ω-Ammoniumalkoxy-hydroxy-phosphoryloxygruppen funktionalisierten Säulenmaterials sämtliche inerten Chromatographie- oder Säulenmaterialien als Materialien geeignet, welche insbesondere nicht mit Blut oder Blutplasma reagieren oder Blut bzw. Blutplasma derart verändern oder kontaminieren, dass das Blut bzw. Blutplasma nach Kontakt mit dem Säulenmaterial einem Patienten nicht mehr injiziert werden kann. Die erfindungsgemäß geeigneten Säulenmaterialien umfassen daher, ohne jedoch hierauf eingeschränkt zu sein: Eupergit®, Polyvinylpyrollidon (PVP), Polysulfon (PS), Polyethersulfon (PES), Polyarylethersulfon (PAES), Polyacrylat, Methacrylat, Methacrylat-Harze wie Poly(methyl methacrylat) (PMMA) und Poly(glycidyl methacrylat) (PGMA), Poly(hydroxy metacrylat), Polystyrol (PS), Polytetrafluorethylen (PTFE), Polyacrylamid, Polyacrolein, Acrylnitril-Butadien-Styrol (ABS), Polyacrylnitril (PAN), Polyurethan (PU), Sepharose®, Acryl-Beads, Agarose, Zellulosematrizen, Polyethylenglykol (PEG), Alginat, Carrageen, Chitin, Stärke, Nitrocellulose, Keramikmatrizen, Glas-Beads und/oder Festphasen-Kieselerde oder Mischungen und/oder Derivate dieser Stoffe. Die Festphasen-Kieselerde-Matrix kann nahezu jede Form von partikulärer Kieselerde umfassen, einschließlich amorpher Kieselerden, wie kolloidale Kieselerde, Kieselgele, präzipitierte Kieselerden, und geräucherte oder pyrogene Kieselerden; mikrokristalline Kieselerden wie Kieselgur; und kristalline Kieselerden wie Quartz. Die Kieselerde sollte eine Partikelgröße im Bereich von ungefähr 45 bis 120 Mesh (entspricht circa 345 μm bis 125 μm) haben, vorzugsweise in dem Bereich von ungefähr 45 bis 60 Mesh (entspricht circa 345 μm bis 212 μm).

Oft wird zur Funktionalsierung mit ω-Phosphonooxyalkylammoniumgruppen und/oder mit ω-Ammoniumalkoxy-hydroxy-phosphoryloxygruppen ein Säulenmaterial verwendet, dass bereits "vorfunktionalsiert" wurde, d.h. mit einer chemischen Gruppe versehen wurde, welche dann wiederum die kovalente Anbindung der ω-Phosphonooxyalkylammoniumgruppen und/oder der ω-Ammoniumalkoxy-hydroxy-phosphoryloxygruppen ermöglicht.

Eine derartige "Vorfunktionalisierung" eines Säulenmaterials wird durch Methoden erzielt, die einem Fachmann wohlbekannt sind (Chin J Chem 2012, 30, 2473; Polym Int 2013, 62, 991). Zusätzlich sind einige bereits "vorfunktionalisierte" Säulenmaterialien kommerziell erhältlich, wie z.B.: Toyopearl® AF-epoxy, Toyopearl® AF-amino, Toyopearl® AFtresyl, TSKgel® tresyl, epoxy-activated Sepharose® 6B (GE Healthcare Life Sciences), CNBr-activated Sepharose® 4 fast flow (GE Healthcare Life Sciences), ECH Sepharose® 4B (GE Healthcare Life Sciences), NHS-activated Sepharose® 4 fast flow (GE Healthcare Life Sciences), terminal vinylsulfone activated Sepharose® 4 fast flow (Affiland), aldehyde Separopore® (Agarose) 4B, ECH Separopore® (Agarose) 4B (Separopore), Agarosen von Sterogene Bioseparations, Inc., z.B. Epoxy-Ultraflow-4 Agarose (Sterogene Bioseparations, Inc.), Epoxy-Ultraflow-6 Agarose (Sterogene Bioseparations, Inc.), Agarosen von *emp* Biotech GmbH, Epoxy-Ultraflow-4 Agarose (*emp* Biotech GmbH), Epoxy-Ultraflow-6 Agarose (*emp* Biotech GmbH), aktivierte Agarosen mit jedwedem Quervernetzungsgrad.

Besonders geeignet ist die erfindungsgemäße Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials für die extrakorporale Entfernung von CRP aus Blut oder Blutplasma zur Reanimation nach Herzstillstand sowie zur Prophylaxe und/oder Behandlung von kardiovaskulären Erkrankungen, wobei es sich bei den kardiovaskulären Erkrankungen insbesondere um Herzinfarkt (Myokardinfarkt), Schlaganfall und Lungenembolie handelt. Ebenso geeignet ist die erfindungsgemäße Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials für die extrakorporale Entfernung von CRP aus Blut oder Blutplasma zur Prophylaxe und/oder Behandlung von Überreaktionen des Immunsystems, wobei die Überreaktionen des Immunsystems ausgewählt werden aus der Gruppe der chronischen Entzündungen, Abstoßungsreaktionen bei Transplantationen und allergischen Reaktionen. Die Verwendung einer Citrat-Lösung gemäß der vorliegenden Erfindung ist daher besonders geeignet zur extrakorporalen Entfernung von CRP aus Blut oder Blutplasma zur Prophylaxe und/oder Behandlung von chronischen Entzündungen ausgewählt aus der Gruppe: Gicht, Osteoarthritis, multiple Sklerose, chronisch-entzündliche Darmerkrankungen wie Morbus Crohn und Colitis ulcerosa, rheumatoide Arthritis, psoriatische Arthritis, Myasthenia gravis, Morbus Basedow (Graves' Disease), autoimmune Thyreoditis wie Hashimoto-Thyreoiditis und Ord-Thyreoiditis, Psoriasis vulgaris, Spondylitis ankylosans (Morbus Bechterew), Goodpasture-Syndrom und idiopathische thrombozytopenische Purpura (ITP), Lupus erythematodes, Sklerodermie, Sjögren-Syndrom, Wegener-Granulomatose (Morbus Wegener), Polymyositis und Dermatomyositis. Unter den oben genannten chronischen Entzündungen befinden sich einige Krankheiten, die unter den Oberbegriff rheumatoide Erkrankungen fallen, wie z.B. rheumatoide Arthritis, psoriatische Arthritis, Myasthenia gravis, Morbus Basedow (Graves' Disease), Hashimoto- Thyreoiditis, Ord-Thyreoiditis, Psoriasis vulgaris, Spondylitis ankylosans (Morbus Bechterew), Goodpasture-Syndrom und idiopathische thrombozytopenische Purpura (ITP), Lupus erythematodes, Sklerodermie, Sjögren-Syndrom, Wegener-Granulomatose (Morbus Wegener), Vasculitis, Polymyalgia rheumatica, Polymyositis und Dermatomyositis.

Citrat ist ein gutes Antikoagulanz, weil es die Aggregation von Blutplättchen sowie die Komplementaktivierung verhindert. Insbesondere bei der Verwendung von Plasmazentrifugen ist das wichtig. Diese benötigen Citrat, da es sonst zur Koagulation kommt. Das ist durch reine Antikoagulation mit Heparin nicht machbar. Zusätzlich wird das Citrat schnell in der Leber im Citratzyklus metabolisiert. Die Patienten werden also nicht zusätzlich systemisch antikoaguliert. Das ist insbesondere bei Infarktpatienten von großem Interesse, da diese im Rahmen ihrer Standardinfarkttherapie ohnehin schon stark antikoaguliert sind (z.B. durch Acetylsalicylsäure, Clopidogrel). Zusätzliche Antikoagulation vergrößert das Blutungsrisiko, was kritisch ist. Außerdem erlaubt einem die Plasmazentrifuge viel geringere Blutflüsse zu realisieren, als ein Membran (Filter)-basiertes Primärtrennsystem. Der übliche bzw. gewünschte Plasmafluss liegt bei circa 30 ml/Minute. Ein Membranprimärtrennsystem benötigt dafür einen Blutfluss von circa 150-200 ml/Minute (Effizienz liegt bei circa 17%). Einer Plasmazentrifuge genügen ≤ 60 ml/Minute Blutfluss (Effizienz circa 87%). Die kleineren Blutflussraten sind insbesondere bei älteren Patienten oder solchen mit wenig Potential viel einfacher zu realisieren. Außerdem wird für ein Membran-basiertes Primärtrennsystem in der Regel ein zentraler Zugang (z.B. Katheter) benötigt, was sehr invasiv ist und bei antikoagulierten Patienten wegen der Blutungsgefahr gefährlich ist, während bei der Verwendung einer Plasmazentrifuge in der Regel ein peripherer Zugang über z.B. eine größere Injektionskanüle genügt, was kein Blutungsproblem darstellt.

## Beschreibung der Figuren

**Fig. 1:** Dargestellt sind die Ergebnisse mehrerer Chromatographieläufe zur Aufreinigung von CRP, wobei in einer halblogarithmischen Darstellung die CRP-Abreicherung in % gegen das Matrixvolumen aufgetragen wurde. Humanes Blutplasma mit einer CRP-Konzentration von 50 mg/L wurde 1:5, 1:10, 1:20 oder 1:50 mit Citrat-Lösung versetzt

und auf eine Chromatographiesäule enthaltend 0,5 g APPC-gekoppelte Epoxy-Ultraflow-4 Agarose (Sterogene) als Matrix aufgetragen. Als Kontrolle diente eine Probe ohne Citratzugabe. Der Säulendurchlauf wurde bei einem Durchfluss von 5, 10, 25, 50, und 75 Matrixvolumina in 1 mL Fraktionen aufgefangen. Anschließend wurde die CRP-Konzentration in den Fraktionen bestimmt und anhand der Ausgangskonzentration an CRP (vor der Auftragung) die CRP-Abreicherung in % bestimmt. Die Datenpunkte stellen Mittelwerte aus mindestens zwei unabhängigen Experimenten dar.

**Beispiele**

Der Begriff **"Matrixvolumen"** (auch als MV abgekürzt), wie hierin verwendet, bezieht sich auf das Volumen des Säulenmaterials, welches in der jeweiligen Affinitätschromatographie verwendet wurde.

**Beispiel 1: Vergleichende Untersuchung zum Einfluss des Zusatzes an Citrat-Lösung auf die affinitätschromatographische Aufreinigung von CRP aus Blutplasma**

Als Ausgangsmaterial wurde zunächst humanes Blutplasma mit einer CRP-Konzentration von circa 50 mg/L unter Verwendung eines 35 µm-Filters filtriert und gegebenenfalls mit Citrat-Lösung versetzt (siehe unten).

<u>Allgemeiner Ablauf der Affinitätschromatographie</u>

Zur Chromatographie wurde eine Niederdruck-Chromatographiesystem (BioLogic™ LP System, Bio-Rad, München Deutschland, Katalognummer 731-8300) zusammen mit einer Säule (Mobicol "Classic", MoBiTec GmbH, Göttingen, Deutschland, Produktnummer M1002) mit einem 35 µM-Filter (MoBiTec GmbH, Produktnummer M523515) verwendet, welche mit 0,5 g APPC-gekoppelter Agarose (Epoxy-Ultraflow-4 Agarose, Sterogene) als Säulenmaterial (1,6 mg APPC/mL) bestückt wurde, d.h. einem Säulenmaterial mit einer Sepharose-Matrix an die das Phosphocholin-Derivat p-Aminophenylphosphocholin (APPC) gekoppelt wurde. Die Säule wurde mit 3 Matrixvolumina (MV) Waschpuffer (0,1 M Tris, 0,2 M NaCl, 2 mM $CaCl_2$, pH 8,0) äquilibriert und der Durchfluss verworfen.

Anschließend wurde die Probe auf die Säule aufgetragen. Durch einen der Säule nachgeschalteten Fraktionssammler (BioFrac™ Fraction Collector, Bio-Rad, Katalognummer 741-0002) wurde der Durchfluss der Säule in Fraktionen von jeweils 1 mL geteilt. Die 1 mL-Fraktionen (auch als Durchflussfraktionen bezeichnet) bei einem Durchfluss von 5 MV, 10 MV, 25 MV, 50 MV, 75 MV und 100 MV wurden gesammelt, während die restlichen Fraktionen zu dem so genannten "Durchflusspool" vereint wurden.

Anschließend wurde die Säule nach dem Probenauftrag mit 25 MV Waschpuffer gewaschen. Es folgte die Elution des an die Säule gebundenen CRPs durch Auftragung von 7,5 MV Elutionspuffers 1 (0,1 M Tris 0,2 M NaCl, 2 mM EDTA, pH 8,0). Hier wurde das Eluat nur dann fraktioniert und aufgefangen, wenn die UV-Absorption über 0,01 betrug.

Anschließend wurden 5 MV Elutionspuffer 2 (Glycinpuffer pH 2,8) zur Regeneration der Säule verwendet. Wenn die Elution durch den Elutionspuffer 1 nicht vollständig verläuft, können sich auch in dieser Fraktion noch Proteine und vor allem CRP befinden. Damit das CRP bei dem sauren pH-Wert des Elutionspuffers 2 von 2,8 nicht denaturiert, wird diesem Eluat Neutralisierungspuffer (3,5 M Tris, pH 9,0) zugesetzt. Schließlich wird die Säule erneut mit 20 MV des Waschpuffers gewaschen.

Der CRP-Gehalt des filtrierten Ausgangsmaterials, des Durchflusspools sowie der gesammelten Fraktionen wurde mittels eines kompetitiven und für humanes CRP spezifischen Immunoassays (siehe z.B. Beispiel 2) durchgeführt.

Ausgehend von der mittleren CRP-Konzentration (auch als mittlere [CRP] bezeichnet) im Ausgangsmaterial wurde dann für jede Fraktion die prozentuale CRP-Abreicherung nach folgender Gleichung bestimmt.

$$CRP - Abreicherung\ (in\ \%) = 100 - \left( \frac{\text{mittlere [CRP] in Fraktion X}}{\text{mittlere [CRP] im Ausgangsmaterial}} \text{ x } 100 \right)$$

Somit konnten anhand der CRP-Konzentration in einer Fraktion, also dem Durchfluss aus der Säule, unter Berücksichtigung der ebenfalls bestimmten CRP-Konzentration im Ausgangsmaterial, Rückschlusse auf die Retention von CRP durch die Säule gezogen werden.

Die Gesamt-CRP-Menge, die auf der Säule verbleibt, kann berechnet werden, indem von der Gesamt-CRP-Menge im Ausgangsmaterial die Gesamt-CRP-Mengen des DurchflussPools und der einzelnen Fraktionen abgezogen werden.

Einfluss von Citrat

Um den Einfluss von Citrat auf die affinitätschromatographische Aufreinigung von CRP aus biologischen Flüssigkeiten, z.B. Blutplasma, mittels eines mit ω-Phosphonooxyalkylammoniumgruppen und/oder mit ω-Ammoniumalkoxy-hydroxy-phosphoryloxygruppen funktionalisierten Säulenmaterials zu untersuchen, wurde das humane Blutplasma in unterschiedlichen Mischungsverhältnissen mit Citrat-Lösung versetzt. Die verwendete Citrat-Lösung war folgendermaßen zusammengesetzt: 7,3 g Zitronensäure (wasserfrei) pro Liter (d.h. 38 mM Zitronensäure), 22,0 g Trinatriumcitrat Dihydrat pro Liter (d.h. 74,8 mM Trinatriumcitrat Dihydrat), ad 1 L mit Wasser. Experimente in denen die Citrat-Lösung zusätzlich 24,5 g Dextrose (Monohydrat) pro Liter (d.h. 123,6 mM Dextrose Monohydrat) enthielt, lieferten identische Ergebnisse.

Hierbei wurden folgende Mischungsverhältnisse untersucht:

**1:50** d.h. 1 Teil Citrat-Lösung + 49 Teile Blutplasma
**1:20** d.h. 1 Teil Citrat-Lösung + 19 Teile Blutplasma
**1:10** d.h. 1 Teil Citrat-Lösung + 9 Teile Blutplasma
**1:5** d.h. 1 Teil Citrat-Lösung + 4 Teile Blutplasma

Als Referenzprobe diente humanes Blutplasma ohne Citratzugabe.

Der Zusatz der Citrat-Lösung zu dem humanen Blutplasma erfolgte vor der Säule unter Verwendung eines Gradientenmischers.

Für die Referenzprobe (kein Citrat-Zusatz) sowie für jede Citrat-Konzentration wurden zwei unabhängige Affinitätschromatographiedurchläufe durchgeführt.

Ergebnisse

In Figur 1 erkennt man, dass bei der Referenzmessung (d.h. ohne Citrat) eine effiziente CRP-Abreicherung aus dem Blut stattgefunden hat. Hierbei lag die prozentuale Abreicherung zu Beginn des Probenauftrages auf die Säule bei über 90% (siehe 5 GV) und sank dann im weiteren Verlauf des Probenauftrages auf knapp unter 50% ab. Ein derartiger Abfall ist damit zu erklären, dass die verwendete Säule mit anhaltendem Probenauftrag immer mehr CRP gebunden hat und damit mehr und mehr gesättigt wird.

Überraschenderweise zeigte sich jedoch bei Mischungsverhältnissen von 1:50 bis 1:5 eine CRP-Abreicherung, die der Abreicherungsleistung unter Referenzbedingungen entspricht (siehe ebenfalls Fig. 1). Entgegen allen Erwartungen konnte also eine effiziente CRP-Entfernung aus Blutplasma unter der erfindungsgemäßen Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials erfolgen.

**Beispiel 2: hu-CRP-ELISA zur Bestimmung der CRP-Konzentration in Proben**

Um die Effektivität einer affinitätschromatographischen Aufreinigung von CRP aus biologischen Flüssigkeiten, z.B. Blutplasma, bestimmen zu können, wurden an entsprechenden Proben Konzentrationsbestimmungen von humanem CRP (hu-CRP) mittels eines Immunoassays (ELISA, *Enzyme Linked Immunosorbent Assay*) durchgeführt.

Hierzu wurden zunächst die Vertiefungen einer Mikrotiterplatte mit einem Antikörper beschichtet, der gegen humanes CRP gerichtet ist, indem pro Vertiefung mit je 100 μl einer Lösung aus polyklonalem anti-human CRP-Antikörper aus Kaninchen (Dako, Hamburg, Deutschland; Produktnummer Q0329), 1:500 in Beschichtungspuffer (0,1 M $NaHCO_3$) verdünnt, für 1 h bei Raumtemperatur inkubiert wurde. Anschließend wurde viermal mit mindestens 250 μl PBST (0,1 % Tween 20 in PBS) gewaschen.

Zur Konzentrationsbestimmung wurde Probenpuffer (5 mM EDTA in PBST) als Nullabgleich sowie eine Standardreihe mit den Konzentrationen von 300, 200, 100, 50, 30, 15 und 8,3 ng CRP/mL (in Probenpuffer) verwendet. Für die Standardreihe wurde ein kommerziell erhältlicher CRP-Standard (*Human Serum C-Reactive Protein Calibrator,* 162 mg CRP/L; Dako, Produktnummer X0923) entsprechend mit Probenpuffer verdünnt.

Darüber hinaus wurden eine Referenzprobe mit hoher CRP-Konzentration (*Human Serum C-Reactive Protein High Control*; Dako, Produktnummer X0926) 1:600 und 1:1500 mit Probenpuffer verdünnt sowie eine Referenzprobe mit niedriger CRP-Konzentration (*Human Serum C-Reactive Protein Low Control*; Dako, Produktnummer X0925) 1:250 und 1:1000 mit Probenpuffer verdünnt und als zusätzliche Referenzproben für den ELISA verwendet. Die eigentlichen zu messenden Proben (also z.B. Ausgangmaterial, Durchflusspool, Durchflussfraktionen) wurden in jeweils drei Verdünnungen (in Probenpuffer) auf die Mikrotiterplatte aufgetragen.

Pro Vertiefung der Mikrotiterplatte wurden jeweils 100 μL aufgetragen, für 1,5 h bei Raumtemperatur und unter

Agitation auf einem Schüttler (600 Umdrehungen pro Minute) inkubiert und anschließend erneut viermal mit mindestens 250 µl PBST gewaschen.

Zur Detektion erfolgte anschließend die Zugabe eines Peroxidase (POD)-markierten Antikörpers (Kaninchen-anti-huCRP-POD-Konjugat, 20 µg/mL Stocklösung, 1:1000 verdünnt) sowie eines kompetitiven polyklonalen anti-human CRP-Antikörpers aus Kaninchen (Dako, Produktnummer Q0329, 1:2000 verdünnt) in Blockierlösung (1 % Casein, 0,9 % NaCl, 0,001 % Thiomersal). Es wurden erneut jeweils 100 µL pro Vertiefung aufgetragen, für 1,5 h bei Raumtemperatur und unter Agitation auf einem Schüttler (600 Umdrehungen pro Minute) inkubiert und anschließend erneut viermal mit mindestens 250 µl PBST gewaschen.

Anschließend erfolgte die Zugabe des Substrates für die Nachweisrektion. Hierzu wurde eine Lösung aus 3,3',5,5'-Tetramethylbenzidin (TMB, 25 mg/mL in DMSO und EtOH) 1:250 in Substratpuffer (0,01 % $H_2O_2$ in 0,2 M Zitronensäure, pH 3,95) verdünnt und hiervon jeweils 100 µl in jede Vertiefung pipettiert. Nach einer Inkubation für 10 bis 15 Minuten im Dunkeln wurde die Nachweisreaktion durch Zugabe von 50 µl Stopplösung pro Vertiefung gestoppt. Anschließend erfolgte die photometrische Detektion durch Bestimmung der Extinktion bei 450 nm (Referenz 655 nm) mithilfe eines Mikrotiterplatten-Lesegeräts (BioRad 680 XR, BioRad, München, Deutschland).

Es erfolgte jeweils eine Doppelbestimmung jeder Mikrotiterplatte zur Eliminierung apparativer Messungenauigkeiten. Zudem wurde jeder Versuchsansatz dreimal wiederholt, um die Reproduzierbarkeit der Ergebnisse zu erhöhen.

In Vorversuchen konnte ein Einfluss der eingesetzten Citrat-Konzentrationen auf die Zuverlässigkeit des hu-CRP-ELISAs ausgeschlossen werden.

Es sei an dieser Stelle darauf hingewiesen, dass natürlich auch andere Methoden zur Bestimmung von CRP angewandt werden können, um die CRP-Konzentration in den zu untersuchenden Proben zu bestimmen.

**Patentansprüche**

1. Verwendung einer Citrat-Lösung zur affinitätschromatographischen Entfernung von CRP aus biologischen Flüssigkeiten mittels eines mit ω-Phosphonooxyalkylammoniumgruppen und/oder mit ω-Ammoniumalkoxy-hydroxy-phosphoryloxygruppen funktionalisierten Säulenmaterials.

2. Verwendung einer Citrat-Lösung gemäß Anspruch 1, **dadurch gekennzeichnet, dass** die ω-Phosphonooxyalkylammoniumgruppen die folgende allgemeine Formel (I) aufweisen

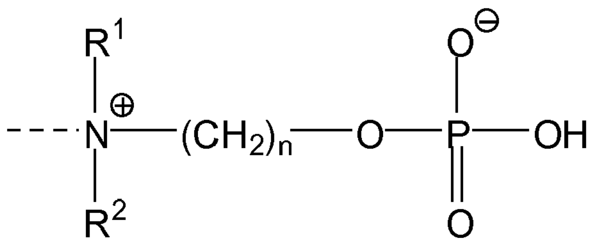

**(I)**

wobei
n ausgewählt wird aus 2 und 3;
$R^1$ und $R^2$ unabhängig voneinander ausgewählt werden aus: -H, $-CH_3$, $-C_2H_5$, $-C_3H_7$, $-C_4H_9$, $-C_5H_{11}$, $-C_6H_{13}$, oder $R^1$ und $R^2$ zusammen mit dem Stickstoffatom mit dem sie verbunden sind einen Heterozyklus bilden können, der ausgewählt wird aus:

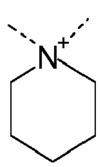 , 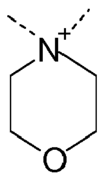 , 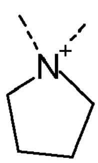 ,

wobei ein oder mehrere Wasserstoffatom(e) durch (ein) Fluoratom(e) ersetzt werden können.

**3.** Verwendung einer Citrat-Lösung gemäß Anspruch 1 oder 2, **dadurch gekennzeichnet, dass** die ω-Ammoniumalkoxy-hydroxy-phosphoryloxygruppen die folgende allgemeine Formel (**II**) aufweisen

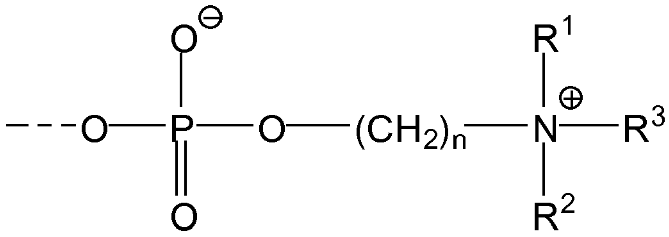

**(II)**

wobei
n ausgewählt wird aus 2 und 3;
$R^1$, $R^2$ und $R^3$ unabhängig voneinander ausgewählt werden aus: -H, $-CH_3$,$-C_2H_5$, $-C_3H_7$, $-C_4H_9$, $-C_5H_{11}$, $-C_6H_{13}$,
oder $R^1$ und $R^2$ zusammen mit dem Stickstoffatom mit dem sie verbunden sind einen Heterozyklus bilden können, der ausgewählt wird aus:

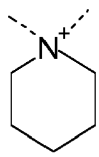 , 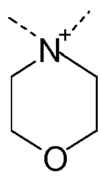 , 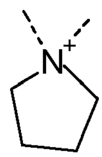 ,

und
$R^3$ ausgewählt wird aus: -H, $-CH_3$, $-C_2H_5$, $-C_3H_7$, $-C_4H_9$, $-C_5H_{11}$, $-C_6H_{13}$, und vorzugsweise -H;
wobei ein oder mehrere Wasserstoffatom(e) durch (ein) Fluoratom(e) ersetzt werden können;

**4.** Verwendung einer Citrat-Lösung gemäß einem der Ansprüche 1 bis 3, wobei die Citrat-Lösung mindestens eine der Citrat-Verbindungen aus der Gruppe umfassend oder bestehend aus Zitronensäure, Natriumdihydrogencitrat, Dinatriumhydrogencitrat, Trinatriumcitrat, Trinatriumcitrat Dihydrat, Kaliumdihydrogencitrat, Dikaliumhydrogencitrat, Trikaliumcitrat, Lithiumdihydrogencitrat, Dilithiumhydrogencitrat, Trilithiumcitrat, Ammoniumdihydrogencitrat, Diammoniumhydrogencitrat, Triammoniumcitrat, Tricalciumdicitrat (Calciumcitrat), Trimagnesiumdicitrat (Magnesiumcitrat) und/oder partielle Citratester enthält.

**5.** Verwendung einer Citrat-Lösung gemäß Anspruch 4, wobei die mindestens eine Citrat-Verbindung ausgewählt wird aus Zitronensäure sowie Citrat-Salzen mit monovalenten Metallkationen.

**6.** Verwendung einer Citrat-Lösung gemäß Anspruch 4 oder 5, wobei die Citrat-Lösung neben der/den Citrat-Verbindung(en) keine zusätzlichen $Ca^{2+}$-Chelatoren enthält.

**7.** Verwendung einer Citrat-Lösung gemäß einem der Ansprüche 1 bis 3, wobei die Citrat-Lösung aus Zitronensäure, Trinatriumcitrat, D-Glucose und Wasser besteht.

**8.** Verwendung einer Citrat-Lösung gemäß einem der Ansprüche 1 bis 7, wobei die Citrat-Lösung eine Gesamtkonzentration an Citrat in einem Bereich von 50 mM bis 200 mM aufweist.

**9.** Verwendung einer Citrat-Lösung gemäß einem der Ansprüche 1 bis 8, wobei die Citrat-Lösung einen pH-Wert in einem Bereich von pH 4,0 bis pH 7,0 aufweist.

**10.** Verwendung einer Citrat-Lösung gemäß einem der Ansprüche 1 bis 9 in einer Verdünnung in einem Bereich von 1:50 bis 1:5 mit der biologischen Flüssigkeit.

**11.** Verwendung einer Citrat-Lösung gemäß einem der Ansprüche 1 bis 10, wobei die Citrat-Lösung als Bindungspuffer bei der affinitätschromatographischen Entfernung von CRP aus biologischen Flüssigkeiten dient.

**12.** Verwendung einer Citrat-Lösung gemäß einem der Ansprüche 1 bis 11, wobei es sich bei den biologischen Flüssigkeiten um Blut oder Blutplasma handelt.

Fig. 1

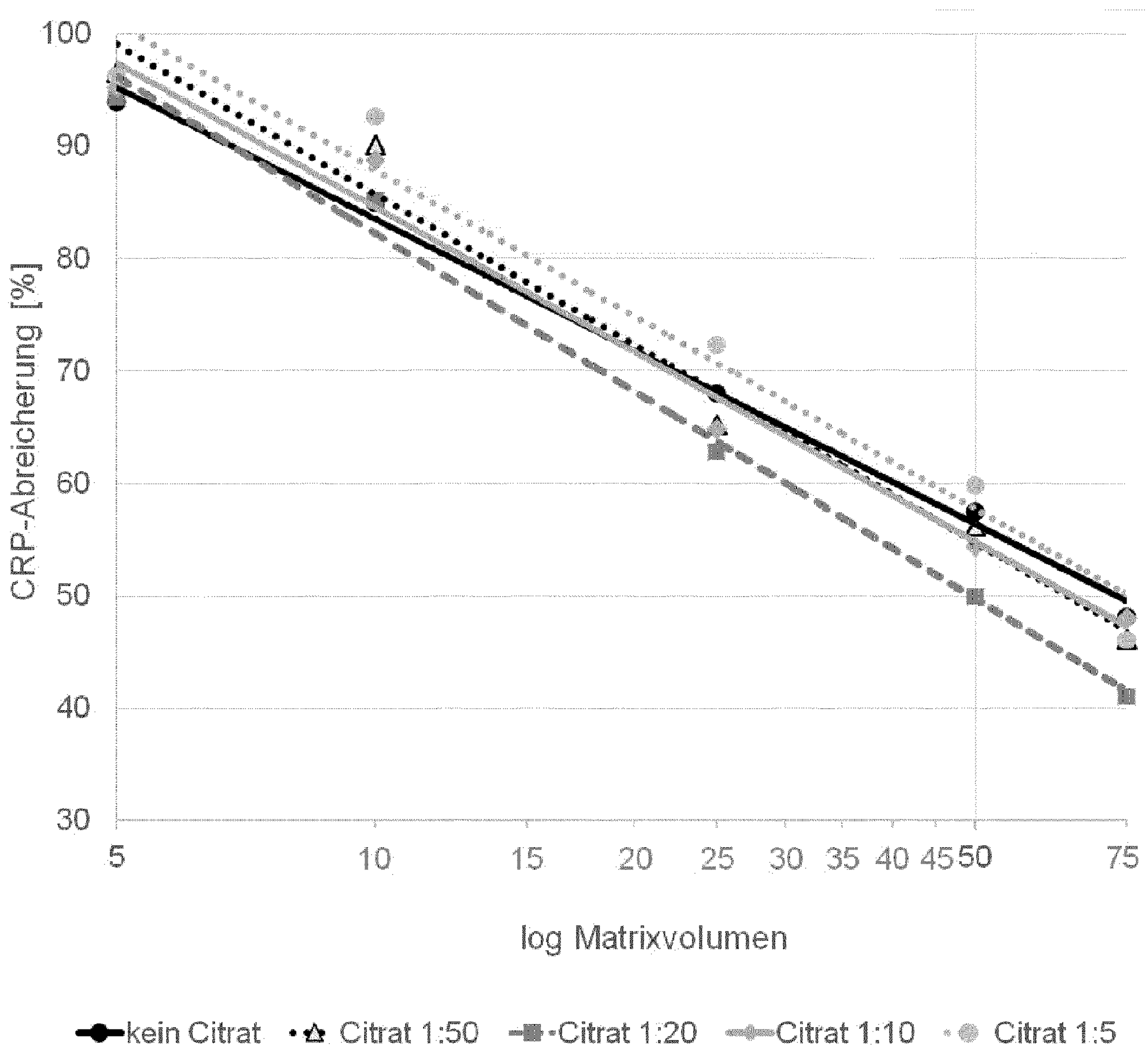
100
90
80
70
60
50
40
30
CRP-Abreicherung [%]
5
10
15
20
25
30
35
40
45
50
75
log Matrixvolumen
kein Citrat
Citrat 1:50
Citrat 1:20
Citrat 1:10
Citrat 1:5

Europäisches Patentamt
European Patent Office
Office européen des brevets

# EUROPÄISCHER RECHERCHENBERICHT

Nummer der Anmeldung
EP 14 19 2831

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2007/225226 A1 (HAMMOND DAVID J [US] ET AL) 27. September 2007 (2007-09-27)<br>* Absatz [0051] - Absatz [0053]; Beispiel 2 *<br>----- | 1-12 | INV.<br>C07K14/47<br>B01D15/12<br>B01D15/38 |
| X | TSENG J ET AL: "Binding of human C-reactive protein (CRP) to plasma fibronectin occurs via the phosphorylcholine-binding site", MOLECULAR IMMUNOLOGY, PERGAMON, GB, Bd. 25, Nr. 8, 1. August 1988 (1988-08-01), Seiten 679-686, XP023682688, ISSN: 0161-5890, DOI: 10.1016/0161-5890(88)90103-4 [gefunden am 1988-08-01] | 1 | |
| Y | * Paragraph "Purification of CRP", page 680 *<br>----- | 1-12 | |
| A | JP S62 36399 A (NIPPON BAIOTESUTO KENKYUSHO KK)<br>17. Februar 1987 (1987-02-17)<br>* Zusammenfassung *<br>----- | 1-12 | |
| A | NUNOMURA W ET AL: "Purification of human C-reactive protein by immunoaffinity chromatography using mouse monoclonal antibody", JOURNAL OF BIOCHEMICAL AND BIOPHYSICAL METHODS, AMSTERDAM, NL, Bd. 21, Nr. 1, 1. Juni 1990 (1990-06-01), Seiten 75-80, XP023454631, ISSN: 0165-022X, DOI: 10.1016/0165-022X(90)90048-H [gefunden am 1990-06-01]<br>* Paragraph "Purification of CRP by immunoaffinity chromatography using mAb (No. 18)" *<br>----- | 1-12 | |

RECHERCHIERTE SACHGEBIETE (IPC)
C07K
B01D

Der vorliegende Recherchenbericht wurde für alle Patentansprüche erstellt

1

| Recherchenort | Abschlußdatum der Recherche | Prüfer |
|---|---|---|
| München | 27. April 2015 | Fourgeaud, Damien |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 14 19 2831

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-04-2015

| Im Recherchenbericht angeführtes Patentdokument | Datum der Veröffentlichung | Mitglied(er) der Patentfamilie | Datum der Veröffentlichung |
|---|---|---|---|
| US 2007225226 A1 | 27-09-2007 | EP 2004273 A2 | 24-12-2008 |
| | | US 2007225226 A1 | 27-09-2007 |
| | | WO 2007111969 A2 | 04-10-2007 |
| JP S6236399 A | 17-02-1987 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004076486 A
- WO 9012632 A
- WO 2007076844 A


### In der Beschreibung aufgeführte Nicht-Patentliteratur


- **YEH.** *Clin Cardiol.,* 2005, vol. 28, 408-412
- **ZOCCALI et al.** *Semin. Nephrol.,* 2005, vol. 25, 358-362
- **NURMOHAMED et al.** *Neth. J. Med.,* 2005, vol. 63, 376-381
- **SOLA et al.** *J. Card. Fail.,* 2005, vol. 11, 607-612
- **PEPYS ; HIRSCHFIELD.** *J. Clin. Invest.,* 2003, vol. 111, 1805-1812
- **SLAGMAN et al.** *Blood Purif.,* 2011, vol. 31, 9
- **BLACK et al.** *Journal of Biological Chemistry,* 2004, vol. 279, 48487
- **THOMPSON et al.** *Structure,* 1999, vol. 7 (2), 169-177
- *Chin J Chem,* 2012, vol. 30, 2473
- *Polym Int,* 2013, vol. 62, 991